# EUROPEAN PATENT SPECIFICATION

(11) **EP 2 388 273 B1**
(45) Date of publication and mention of the grant of the patent: **05.07.2017**
(21) Application number: 11167183.0
(22) Date of filing: 23.10.2006
(51) Int. Cl.: C07K 16/04, A61K 39/395, C07K 16/00, C07K 16/28

(54) **Antibodies with enhanced antibody-dependent cellular cytoxicity activity, methods of their production and use**
Antikörper mit verbesserter antikörperabhängiger Zelltoxizitätsaktivität sowie Herstellungs- und Verwendungsverfahren dafür
Anticorps avec activité cytotoxique cellulaire améliorée dépendant des anticorps, leurs procédés de production et utilisation

(30) Priority: 21.10.2005 US 729054 P
(43) Date of publication of application: 23.11.2011
(62) Divisional of application: 06844220.1
(73) Proprietor: LFB USA, Inc., Framingham, MA 01702 (US); Genzyme Corporation, Cambridge, MA 02142 (US)
(72) Inventor: Schindler, Daniel, Newton Upper Falls, MA 02464 (US); Meade, Harry M, Newton, MA 02158 (US); Edmunds, Timothy, Bolton, MA 01740 (US); McPherson, John, Hopkinton, MA 01748 (US)
(74) Representative: Chajmowicz, Marion

(56) References cited:
- EP-A- 1 400 171
- WO-A-2004/009618
- WO-A-2004/050847
- WO-A-2006/088447
- WO-A-2006/088464
- KOLB A F ET AL: "Virus-Neutralizing Monoclonal Antibody Expressed in Milk of Transgenic Mice Provides Full Protection against Virus-Induced Encephalitis", JOURNAL OF VIROLOGY, THE AMERICAN SOCIETY FOR MICROBIOLOGY, US, vol. 75, no. 6, March 2001 (2001-03), pages 2803-2809, XP002979585, ISSN: 0022-538X
- LIU A ET AL: "A COMPARISON OF HERPES SIMPLEX VIRUS SPECIFIC ANTIBODIES FOUND IN HUMAN MILK AND SERUM", PEDIATRIC RESEARCH, vol. 31, no. 6, 1992, pages 591-595, XP008076550, ISSN: 0031-3998

### SEQUENCE LISTING

<110> GTC Biotherapeutics Inc.
<120> Antibodies with enhanced antibody-dependent cellular cytotoxicity activity, methods of their production and use
<130> PC780656EPA
<150> US 60/729,054
   <151> 2005-10-21
<150> PCT/US2006/041656
   <151> 2006-10-23
<150> EP06844220.1
   <151> 2006-10-23
<160> 2
<170> PatentIn version 3.3
<210> 1
   <211> 28
   <212> DNA
   <213> Artificial
<220>
   <223> synthetic oligonucleotide
<400> 1
   agggtaccaa gcttgaaatc aaacgaac 28
<210> 2
   <211> 48
   <212> DNA
   <213> Artificial
<220>
   <223> synthetic oligonucleotide
<400> 2
   aagggtccgg atcctcgagg atcctaacac tctcccctgt tgaagctc 48

## Description

### FIELD OF THE INVENTION

The present invention relates to antibodies with enhanced antibody-dependent cellular cytotoxicity (ADCC) activity, methods of their production as well as methods of their use.

### BACKGROUND OF THE INVENTION

Monoclonal antibodies are an important weapon in the disease therapy armory. For example, ADCC is a major contributor to the effectiveness of anti-cancer antibodies. The key receptor mediating ADCC is the FcγIIIa (CD16) receptor, a low affinity receptor for IgG expressed on natural killer (NK) cells. Mice deficient for this receptor have a significantly less anti-tumor response to rituximab (Clynes et al., 2000, Nat. Med 6: 443-446). The FcγIIIa receptor has a role in mediating tumor cytotoxicity in mice (Clynes et al, 1998, PNAS 95: 652-656). A polymorphism of the FCGR3A gene coding for the FcγIIIa receptor, which has a valine at position 158 instead of a phenylalanine, binds IgG more tightly and confers enhanced ADCC activity upon NK cells (Wu et al., 1997, J Clin Invest 100: 1059-1070). This genotype of the FCGR3A gene, FCGR3A-158V/V, has a positive effect on patient response to rituximab in non-Hodgkin's lymphoma (Cartron et al., 2002, Blood 99: 754-758), Waldenstrom's macroglobulinemia (Treon et al., 2005, J Clin Oncol 23: 474-481), and the autoimmune disease systemic lupus erythematosus (SLE) (Anolik et al., 2003, Arthritis Rheum 48, 455-459). The same polymorphism has also been correlated with a better biological response in Crohn's disease to another antibody, infliximab (Louis et al., 2004, A Phar Ther 19: 511-519). These findings exemplify that ADCC plays a significant role in the therapeutic action of monoclonal antibodies in humans. Increasing the ability of therapeutic antibodies to facilitate ADCC activity, therefore, has potential therapeutic value.

Human antibodies have two glycosylation sites, one on each of the identical heavy chains. Early experiments using inhibitors of glycosylation and carbohydrate processing found that antibodies produced in inhibited hybridoma clones have enhanced ADCC activity (Rothman, 1989). Additional work showed that both the glycosylation state and ADCC activity of an antibody are affected by the cell line in which the antibody was produced (Lifely, 1995). Several research groups have demonstrated that antibodies lacking the 1,6-fucose on their heavy chain glycosylation, have enhanced binding affinity to the FcγRIII receptor and increased ADCC activity (Shields et al., 2002; Shinkawa et al, 2002). In addition, a correlation between binding affinity to the FcγRIII receptor and ADCC activity has been established (Okazaki, 2004; Dall'Ozzo, 2004).

Cell lines have been generated that are deficient for 'FUT8', alpha-1,6 fucosyltransferase, which catalyzes the transfer of this fucose. These knock-out cells can be used to produce antibodies with higher ADCC activity. For instance, a Chinese hamster ovary (CHO) cell deficient in FUT8 has been established (Yamane-Ohnuki et al., 2004). Small interfering RNA (siRNA) has also been used to block the expression of the FUT8 gene (Mori et al., 2004). A rat cell line has been used to make antibodies with increased ADCC activity (Niwa et al., 2004, 2005). In addition to its higher activity, low fucose IgG1 is independent of the FcγRIII polymorphism, thereby lacking the difference seen with trastuzumab or rituximab in the wild-type cell (Niwa et al., 2004b, Vol 10 Clin Canc Res).

Even though antibodies have been produced previously in a variety of expression systems, the enhanced ADCC activity of antibodies produced in mammalian mammary epithelial cells, such as in the mammalian mammary epithelial cells of a transgenic animal engineered to secrete the antibody in its milk, has not been previously recognized.

### SUMMARY OF THE INVENTION

It has been found that antibodies produced in mammalian mammary epithelial cells, such as in the mammalian mammary epithelial cells of a transgenic animal engineered to secrete antibodies in its milk, have enhanced ADCC activity and increased binding to CD16 compared to cell culture-derived material. Studies on the glycosylation of the milk-derived antibodies reveal lower fucose contents in the milk-derived antibodies. Therefore, antibodies and compositions thereof are provided herein. The antibodies provided are those produced in mammalian mammary epithelial cells. Also provided are methods of producing the antibodies as well as methods of their use.

The invention is as defined in the claims.

The antibodies with enhanced ADCC activity are produced by expressing the antibodies in mammalian mammary epithelial cells. Mammalian mammary epithelial cells are those of a transgenic non-human mammal engineered to express antibodies in its milk.

In one aspect of the invention, therefore, a composition comprising mammary epithelial cell-derived antibodies (or an antibody) wherein the antibodies have enhanced antibody-dependent cellular cytotoxicity (ADCC) activity is provided. In another aspect of the invention a composition comprising milk-derived antibodies (or an antibody) is provided, wherein the milk-derived antibodies have enhanced antibody-dependent cellular cytotoxicity (ADCC) activity. In one embodiment the ADCC activity of the antibodies is at least two-fold higher than the ADCC activity of cell culture-derived antibodies. In another embodiment the ADCC activity of the antibodies is at least three-fold, four-fold, five-fold, seven-fold or tenfold higher than the ADCC activity of cell culture-derived antibodies. In one embodiment the milk from which the milk-derived antibodies are obtained is the milk from a non-human transgenic mammal engineered to express the antibody.

According to an aspect of the invention the binding of the Fc region of IgG1 antibodies is enhanced if the antibody lacks the core fucose found attached to the Asn297 glycosylation site in the Fc region (e.g., a fucose linked to the base of the biannetennary structure). The antibodies in some aspects lack this core fucose. Milk-produced monoclonal antibodies in other aspects are predominantly high mannose and hybrid structures. Generally, the antibodies can bind more tightly to the FcRIII receptor and can result in enhanced ADCC.

According to the invention the antibodies have been modified to have a glycosylation pattern as provided herein and as a result have enhanced ADCC activity. The modification is the result of producing the antibody so that it is expressed in mammalian mammary epithelial cells as provided herein.

The antibodies of the compositions provided can be homogeneous or heterogeneous with respect to their glycosylation.

In one embodiment at least one chain of the antibodies have been modified so that it does not contain fucose. In another embodiment one chain of the antibodies has been modified so that it does not contain fucose. In still another embodiment the fucose that at least one chain of the antibodies do not contain is 1,6-fucose. In another embodiment one or at least one chain of the antibodies have been modified to not contain fucose but the antibodies have also been modified to contain an oligomannose or an additional oligomannose. The chain that contains the oligomannose can be the same chain that does not contain fucose but is not necessarily so. In another embodiment the amino acid sequence of the antibodies have been modified and then expressed in mammalian mammary epithelial cells. In one embodiment the amino acid sequence of the antibodies have been modified. In another embodiment the amino acid sequence of the antibodies, such as anti-CD 137 antibodies, has an amino acid substitution of Asn297. In a further embodiment the substitution is with an amino acid that will not be fucosylated. In another embodiment the Asn297 is substituted with Gln.

In another embodiment the antibodies provided have been modified to contain an oligomannose or an additional oligomannose. In another embodiment the antibodies have been modified so that at least 30% of the antibodies contain at least one oligomannose. In another embodiment the antibodies have been modified so that at least 40%, 50%, 60%, 70%, 80%, 90% or more of the antibodies contain at least one oligomannose. In a further embodiment the antibodies have been modified so that less than 50%, 40%, 30%, 20%, 10%, or fewer of the antibodies do not contain fucose on one or at least one chain. In still a further embodiment the antibodies have been modified so at least 40%, 50%, 60%, 70%, 80%, 90% or more of the antibodies contain at least one oligomannose and less than 50%, 40%, 30%, 20%, 10%, or fewer of the antibodies contain fucose on one or at least one chain.

In another embodiment the carbohydrates of the antibodies have been modified to exhibit a high mannose glycosylation pattern. In still a further embodiment the antibodies have been modified so that at least one chain of the antibodies contain an oligomannose and is non-fucosylated. In yet another embodiment the antibodies have been modified so that the major carbohydrate of the antibodies is non-fucosylated. In one embodiment the major carbohydrate is a non-fucosylated oligomannose. In another embodiment the major carbohydrate is a non-fucosylated Man5. In yet another embodiment the antibodies have been modified so that less than 40% of the carbohydrates of the antibodies contain fucose. In still another embodiment the antibodies have been modified so that less than 30%, 20%, 10% or fewer of the carbohydrates of the antibodies contain fucose. In one embodiment the fucose is 1,6-fucose. In another embodiment the antibodies have been modified so that at least 60% of the carbohydrates of the antibodies are a non-fucosylated oligomannose and less than 40% of the carbohydrates of the antibodies are fucose-containing. In a further embodiment the antibodies have been modified so that 63% of the carbohydrates of the antibodies are a non-fucosylated oligomannose, 16% of the carbohydrates of the antibodies are a core fucose-containing G1F and 21% of the carbohydrates of the antibodies are a core fucose-containing G2F.

In another aspect of the invention the antibodies with enhanced ADCC activity are not in fact modified by the means provided herein but are antibodies selected for the above glycosylation patterns.

In one embodiment the antibodies are monoclonal antibodies. In another embodiment the antibodies are polyclonal antibodies. In a further embodiment the antibodies are chimeric antibodies, humanized antibodies or fully human antibodies. In another embodiment the antibodies are full-length antibodies. In yet another embodiment the antibodies are full-length single chain antibodies. In still another embodiment the full-length antibodies comprise a heavy chain and a light chain. In a further embodiments the antibodies are antibody fragments. In still a further embodiment the antibody fragments are part of Fc fusion polypeptides.

In another aspect of the invention the antibodies are encoded by a transgene DNA construct comprising an Fc region containing N-linked oligosaccharides. According to the invention, the antibodies are produced in the milk of a non-human transgenic animal. In still another embodiment the antibodies have a glycosylation pattern as described herein.

In one embodiment the antibodies are of the isotype IgG, IgA or IgD. In a further embodiment the antibodies are of the isotype IgG. In another embodiment the antibodies are of the isotype IgG1 or IgG2. In a further embodiment the antibodies are antibody fragments. In another aspect of the invention humanized versions of the antibodies of the invention with improved characteristics (e.g., ADCC characteristics) are provided.

The antibodies can be directed against any antigen. In one embodiment the antibodies are directed against CD3, CD4, CD5, CD8, CD14, CD15, CD16, CD19, CD20, CD21, CD22, CD23, CD25, CD32B, CD30, CD33, CD37, CD38, CD40, CD40L, CD46, CD52, CD54, CD59, CD74, CD80, CD126, CD138, CD137 or GPIIbIIIa. In another embodiment the antibodies are directed against HM1.24, HLA-DR, MUC1, tenascin, PIGF, VEGF, an oncogene, an oncogene product, a necrosis antigen, 17-A1 antigen, IL-2, T101, TAC, IL-6, TRAIL-R1, GD3 ganglioside or TRAIL-R2. In one embodiment the antibodies are anti-CD 137 antibodies.

In any of the compositions and methods provided herein the antibodies are anti-CD 137 antibodies.

In one embodiment the compositions comprising the antibodies provided can further comprise a pharmaceutically acceptable carrier. In another embodiment the compositions provided can further comprise an additional therapeutic agent. In one embodiment the additional therapeutic agent is an anti-cancer agent or an immunomodulatory agent.

It is also described pharmaceutical compositions which comprise an amount of a transgenic protein of interest, a prodrug thereof, or a pharmaceutically acceptable salt of said compound or of said prodrug and a pharmaceutically acceptable vehicle, diluent or carrier.

The compositions provided can be used in a number of methods of treatment. A method of treating a subject is described, comprising administering to a subject in need thereof a composition provided herein in an amount effective to enhance ADCC in the subject. Another method of treating a subject is described, comprising administering to a subject a composition provided in an amount effective to treat a disease the subject has or is at risk of having is provided. In one embodiment the disease is cancer. Specific indications against which the antibodies described herein could provide beneficial therapeutic effects, in some embodiments, include treatment of solid tumors; melanomas; as well as carcinomas of the breast, colon, ovaries, kidney, prostate and lung. It is thought, without being limiting, that the treatment could be effective because the antibodies will help provide effective immunomodulatory treatment.

Specific targets can, therefore, also include anti-CD3 antibodies (e.g., non-Hodgkin's Lymphoma; autoimmune disease - SLE), anti-CD16 antibodies (e.g., FcRIII), anti-CD19 (e.g., non-Hodgkin's Lymphoma), anti-CD20, anti-CD32B antibodies (e.g., FcRIIB and allergy), anti-CD30 (e.g., Hodgkin's Disease), anti-GPIIbIIIa (e.g., Thrombosis), anti-TNF- α (e.g., for rheumatoid arthritis and Crohn's Disease), anti-TEM antibodies for tumor endothelial markers, (e.g., for the control of angiogenesis - anticancer), etc.

In another embodiment the disease is lymphoproliferative disease. In a further embodiment the disease is an autoimmune disease. In another embodiment of the invention the ADCC antibodies of the invention are effective in the treatment of autoimmune-derived encephalo-myelitis, systemic lupus erythematosis, as well as other disease states in which anti-CD137 can confer some benefit.

In one embodiment the amount of the antibody administered to the subject is about 0.01 mg/kg/day to about 50 mg/kg/day.

In another embodiment the subject is further administered an additional therapeutic agent. In one embodiment the additional therapeutic agent is an anti-cancer agent. In another embodiment the additional therapeutic agent is an immunomodulator. In one embodiment the immunomodulator is IL-1, IL-2, IL-3, IL-6, IL-10, IL-12, IL-18, IL-21, an interferon, paclitaxel, TNF-α or a combination thereof. In another embodiment the subject is furthered administered IL-2. In another embodiment the subject is furthered administered IL-12. In a further embodiment the subject is further administered an anti-cancer agent and an immunomodulator. In one embodiment the subject is further administered an immunomodulator and trastuzumab. In yet another embodiment the subject is furthered administered IL-21 and trastuzumab. In one embodiment an anti-CD 137 mammary epithelial cell-derived antibody is used in combination with IL-21 and trastuzumab. In another embodiment the anti-CD137 antibody is a high mannose antibody lacking a core fucose. Therefore, the antibodies in some embodiments are used with an immune modulator that is effective in shrinking solid tumors and preventing their recurrence. In other embodiments the antibodies are used with other anti-cancer therapeutic agents such as IL-21, IL-12 and/or trastuzumab.

The subject can be any subject in which ADCC activity is desirable. In one embodiment the subject is a human. In another embodiment the subject is a dog, cat, horse, cow, pig, sheep, goat or primate.

Methods for producing antibodies with enhanced ADCC activity by modifying the antibodies are also provided. These methods comprise modifying the glycosylation of the antibodies by producing the antibody in mammalian mammary epithelial cells. In one embodiment the mammalian mammary epithelial cells are of a non-human mammal engineered to express the antibody in its milk. In yet another embodiment the mammalian mammary epithelial cells are mammalian mammary epithelial cells in culture.

It is described a method for the production of a transgenic antibody, and variants and fragments thereof, the process comprises expressing in the milk of a transgenic non-human mammal a transgenic antibody encoded by a nucleic acid construct. In one embodiment the method for producing the antibodies of the invention comprises:
(a) transfecting non-human mammalian cells with a transgene DNA construct encoding a desired transgenic antibody;
(b) selecting cells in which said transgene DNA construct has been inserted into the genome of the cells; and
(c) performing a first nuclear transfer procedure to generate a non-human transgenic mammal heterozygous for the desired transgenic antibody and that can express it in its milk.

In another embodiment the method comprises:
(a) providing a non-human transgenic mammal engineered to express an antibody,
(b) expressing the antibody in the milk of the non-human transgenic mammal; and
(c) isolating the antibodies expressed in the milk.

In other embodiments the methods further comprise steps for inducing lactation and/or steps for determining the ADCC activity of the antibodies obtained. In still other embodiments the methods further comprise additional isolation and/or purification steps. In yet other embodiments the methods further comprise steps for comparing the ADCC activity of the antibodies obtained with antibodies produced in cell culture. In further embodiments the methods further comprise steps for comparing the ADCC activity of the antibodies obtained to antibodies produced by non-mammary epithelial cells. Such cells can be cells of a cell culture.

The antibodies can be obtained, in some embodiments, by collecting the antibodies from the milk of a transgenic animal produced as provided herein or from an offspring of said transgenic animal.

In some embodiments the construct encoding the desired antibody (or antibody fusion polypeptide) is actuated by at least one beta-casein promoter. In other embodiments the non-human transgenic mammal is an ungulate. In still other embodiments the non-human transgenic mammal is a goat.

In some embodiments the antibodies produced by the transgenic mammal is produced at a level of at least 1 gram per liter of milk produced.

In another aspect a method for enhancing the ADCC activity of an antibody, comprising modifying the glycosylation of the antibody by the methods provided herein is provided. In one embodiment the antibody is modified such that at least one chain of the antibody does not contain fucose. In another embodiment the antibody is modified such that one chain of the antibody does not contain fucose. In yet another embodiment the antibody is modified such that it contains an oligomannose or an additional oligomannose. In still another embodiment the antibody is modified such that it contains an oligomannose or an additional oligomannose.

In yet another embodiment the antibodies are modified such that the carbohydrates of the antibodies exhibit a high mannose glycosylation pattern. In one embodiment the antibodies are modified such that at least one chain of the antibodies is oligomannose-containing and non-fucosylated. In another embodiment the antibodies are modified such that one chain of the antibodies is oligomannose-containing and non-fucosylated. In a further embodiment the antibodies are modified such that the major carbohydrate of the antibodies is non-fucosylated. In one embodiment the major carbohydrate is a non-fucosylated oligomannose. In another embodiment the major carbohydrate is a non-fucosylated Man5. In yet a further embodiment the antibodies are modified such that less than 40% of the carbohydrates of the antibodies contain fucose. In one embodiment the antibodies are modified such that less than 30%, 20%, 10% or fewer of the carbohydrates of the antibodies contain fucose. In still a further embodiment the antibodies are modified such that at least 30% of the antibodies have at least one oligomannose. In one embodiment the antibodies are modified such that at least 40%, 50%, 60%, 70%, 80%, 90% or more of the antibodies have at least one oligomannose. In yet a further embodiment the antibodies are modified such that at least 40%, 50%, 60%, 70%, 80%, 90% or more of the antibodies contain at least one oligomannose and less than 50%, 40%, 30%, 20%, 10%, or fewer of the antibodies contain fucose on one or at least one chain. In another embodiment the antibodies are modified such that at least 60% of the carbohydrates of the antibodies are a non-fucosylated oligomannose and less than 40% of the carbohydrates of the antibodies are fucose-containing. In a further embodiment the antibodies are modified such that 63% of the carbohydrates of the antibodies are a non-fucosylated oligomannose, 16% of the carbohydrates of the antibodies are a core fucose-containing G1F and 21% of the carbohydrates of the antibodies are a core fucose-containing G2F.

In yet a further aspect a method for producing an antibody, comprising collecting the antibody from the milk of a transgenic non-human mammal engineered to express the antibody in its milk and determining the ADCC activity of the antibody is provided. In one embodiment the method further comprises comparing the ADCC activity of the collected antibody with the ADCC activity of the antibody expressed in cell culture. In another embodiment the transgenic non-human mammal is a goat, sheep, bison, camel, cow, pig, rabbit, buffalo, horse, rat, mouse or llama.

In another aspect a method for producing an antibody, comprising collecting the antibody from mammary epithelial cells engineered to express the antibody and determining the ADCC activity of the antibody is provided. In one embodiment the method further comprises comparing the ADCC activity of the collected antibody with the ADCC activity of the antibody expressed in non-mammary epithelial cells.

In still a further aspect a method is provided comprising obtaining antibodies as in the methods provided herein and comparing the ADCC activity of the antibodies to another set of antibodies. In one embodiment the other set of antibodies are antibodies obtained from cell culture. In another embodiment the other set of antibodies are antibodies obtained from non-mammary epithelial cells in culture. In a further embodiment the other set of antibodies are antibodies obtained from mammary epithelial cells in culture. In yet a further embodiment the other set of antibodies are antibodies obtained from body fluid or tissue. In still a further embodiment the other set of antibodies are antibodies obtained from the milk of a mammal. In one embodiment the mammal is a non-human transgenic mammal engineered to express the other set of antibodies in its milk.

### BRIEF DESCRIPTION OF THE DRAWINGS

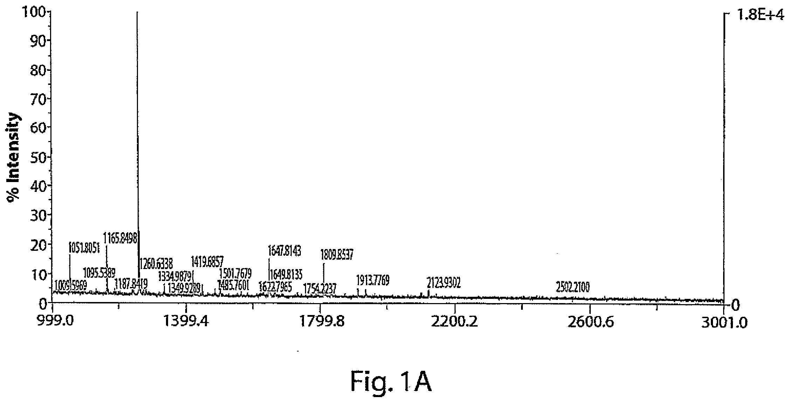

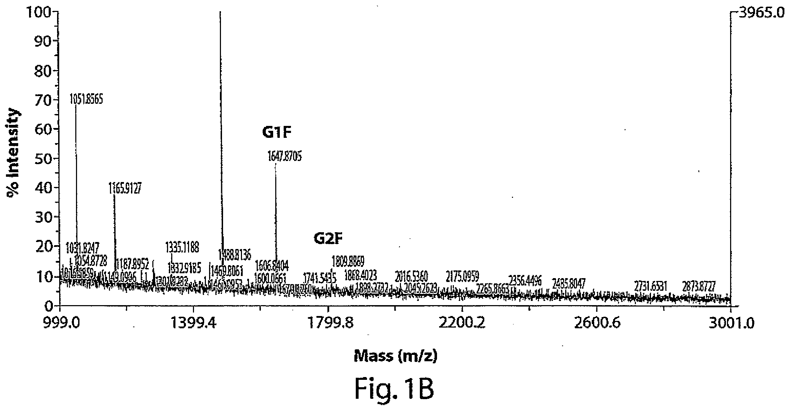

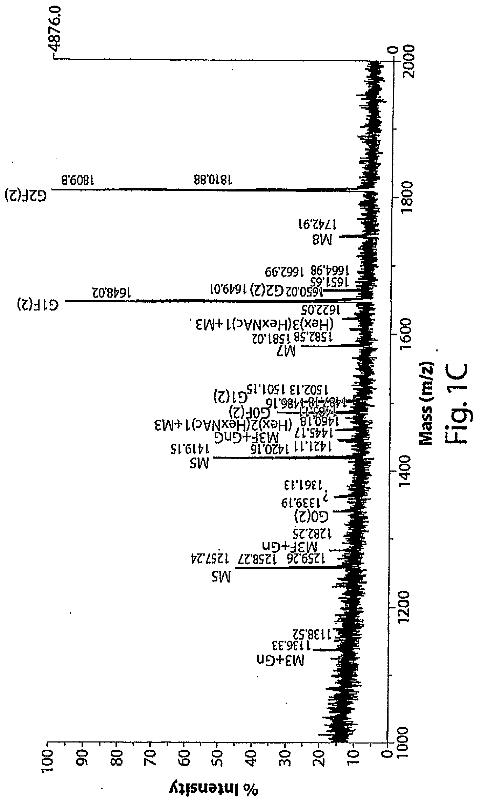

**Fig. 1** provides MALDI-TOF spectra of N-linked oligosaccharides of glycosylated anti-CD 137 antibody. Glycans were released by PNGase F. **Fig. 1A** provides a spectrum for mouse milk-derived antibody; **Fig. 1B** provides a spectrum for cell culture-derived antibody; **Fig. 1C** provides a spectrum for goat-milk derived antibody.

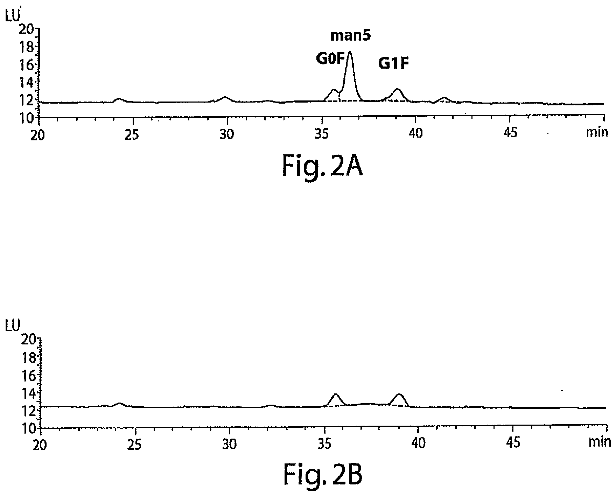

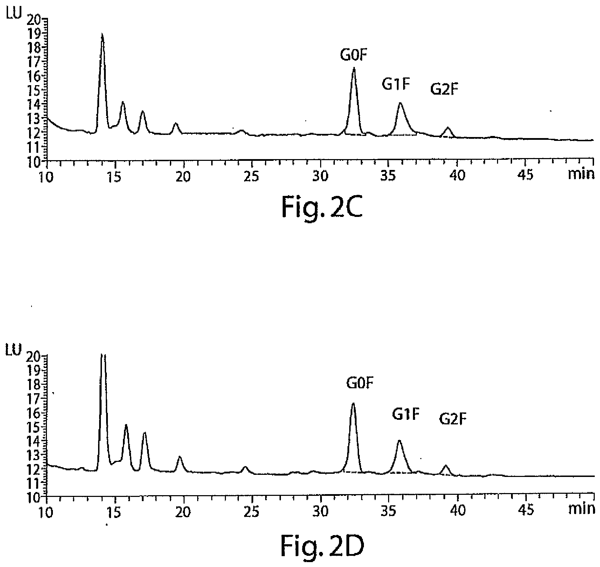

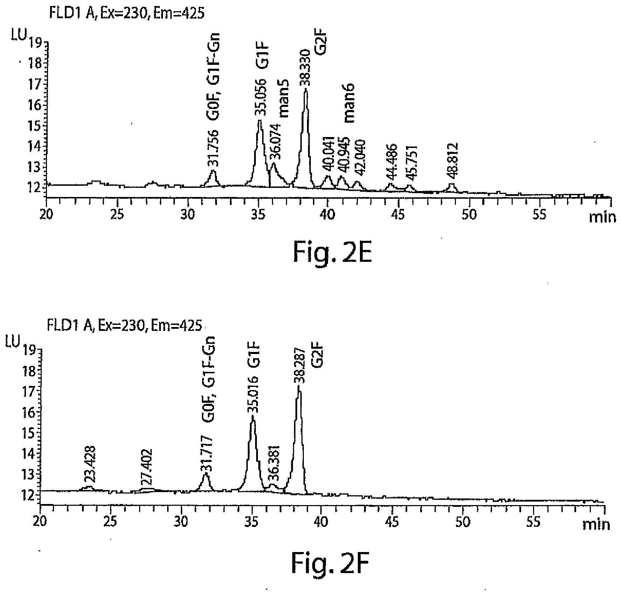

**Fig. 2** provides a comparison of oligosaccharide maps obtained by HPLC with fluorescence detection (2-AA labeled) of released oligosaccharides from glycosylated antibodies. **Fig. 2A** provides a comparison with mouse milk-derived antibody; **Fig. 2B** provides a comparison with mouse milk-derived antibody treated with Endo H; **Fig. 2C** provides a comparison with cell culture-derived antibody; **Fig. 2D** provides a comparison with cell culture-derived antibody treated with Endo H; **Fig. 2E** provides a comparison with goat milk-derived antibody; **Fig. 2F** provides a comparison with goat milk-derived antibody treated with Endo H.

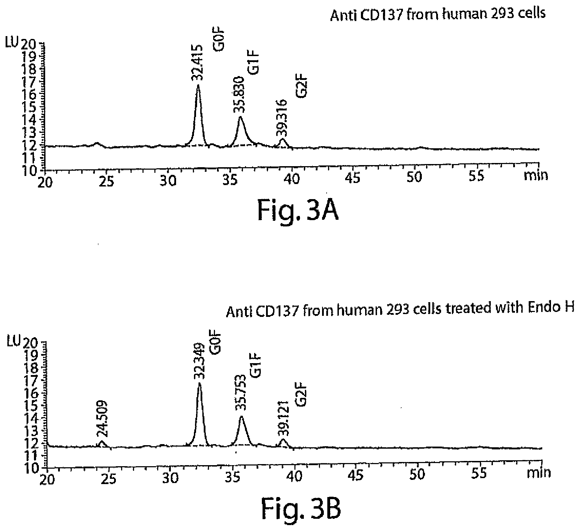

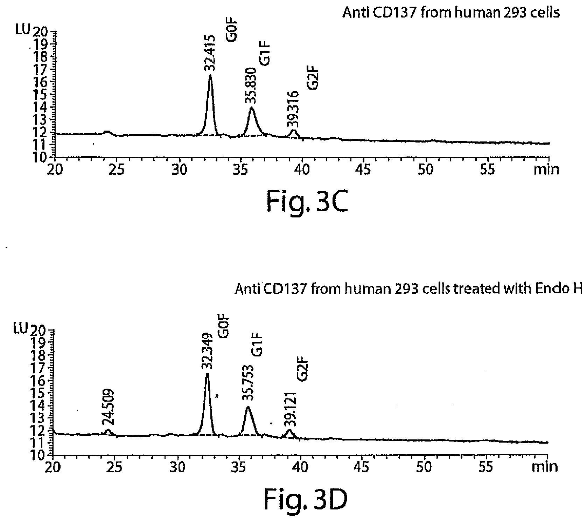

**Fig. 3** illustrates the Endo H sensitivity of major oligosaccharides released from mouse milk derived anti-CD137 antibody. The sample from **Fig. 2** was treated overnight with Endo H. **Fig. 3A** provides the results for mouse milk-derived antibody; **Fig. 3B** provides the results for mouse milk-derived antibody treated with Endo H; **Fig. 3C** provides the results for cell culture-derived antibody; **Fig. 3D** provides the results for cell culture-derived antibody treated with Endo H.

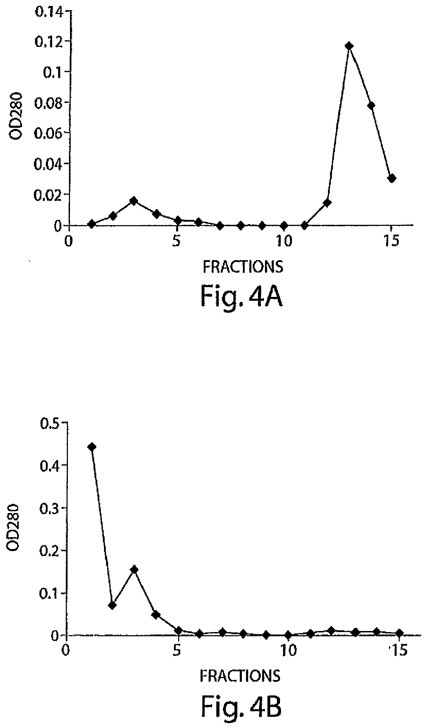

**Fig. 4** shows concanavalin A (Con A) binding of antibodies. Protein A purified antibody was loaded on a Con A column and eluted with alpha methyl mannosidase-containing buffer. Fractions were monitored for absorbance at 280 nm. **Fig. 4A** shows the results for glycosylated anti-CD 137 antibody on a Con A column; **Fig. 4B** shows the results for non-glycosylated anti-CD 137 antibody on a Con A column.

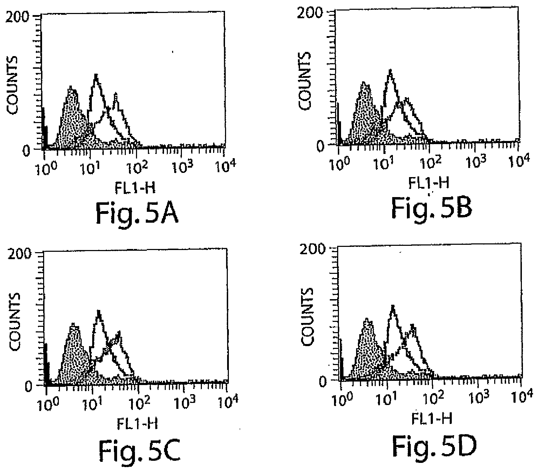

**Fig. 5** demonstrates the binding of antibodies to 786-O cells. Binding of each antibody was measured by FACS analysis and overlaid on a negative control antibody (anti-2,4-dinitrophenol (DNP)) and anti-HER2. **Fig. 5A** shows the results with mouse milk-derived aglycosylated antibody; **Fig. 5B** shows the results with mouse milk-derived glycosylated antibody; **Fig. 5C** shows the results with cell culture-derived glycosylated antibody; **Fig. 5D** shows the results with goat milk-derived glycosylated antibody.

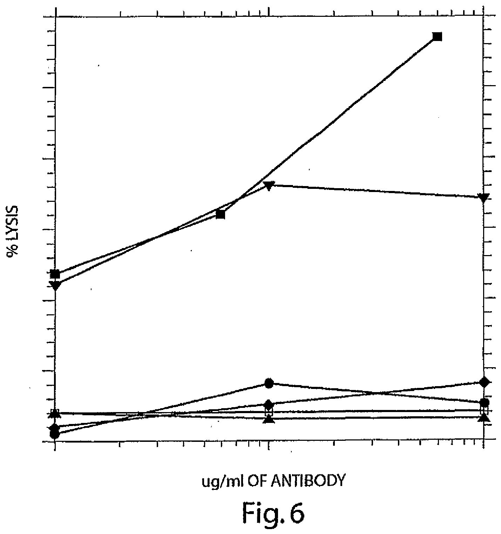

**Fig. 6** illustrates the enhanced killing of 786-O cells by milk-derived antibody in an ADCC assay. Squares: mouse milk-derived; inverted triangles: goat-derived; diamonds: cell culture-derived; circles: aglycosylated mouse milk-derived; open squares: trastazumab; triangles: anti-DNP antibody.

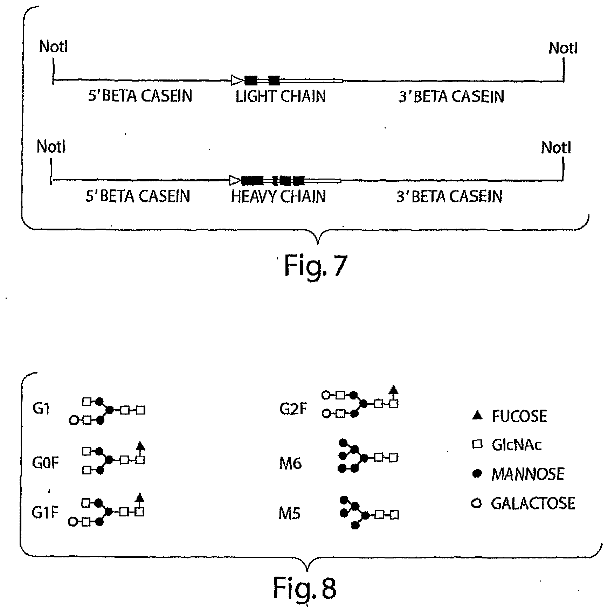

**Fig. 7** provides a general schematic of transgene constructs for milk expression of antibodies. The gene of interest replaces the coding region of caprine beta-casein, a milk specific gene. The 6.2 kb promoter region is linked to the coding regions of either the H or L IgG chains, followed by untranslated caprine beta casein 3' sequences and downstream elements. Black boxes: H and L exons; striped boxes: genomic introns; arrows: direction of transcription.
**Fig. 8** provides a breakdown of the carbohydrates present or absent on antibodies produced by transgenic animals.

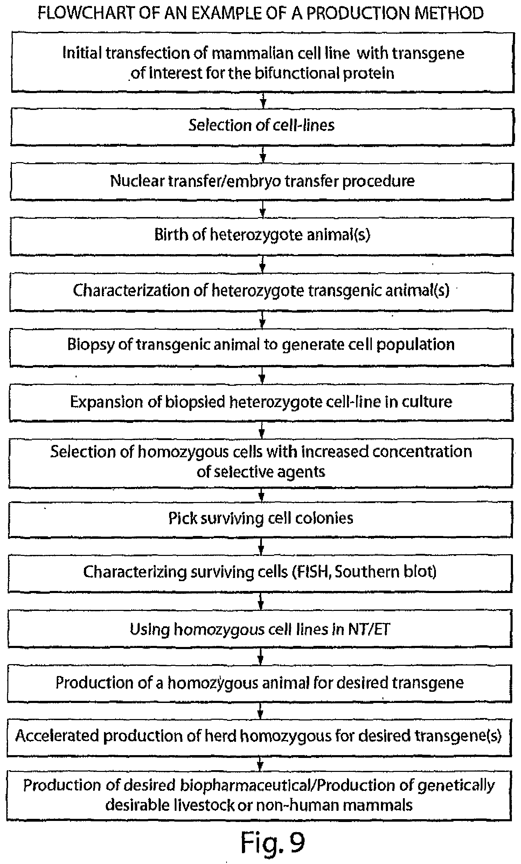

**Fig. 9** provides a general schematic for an example of a production method.

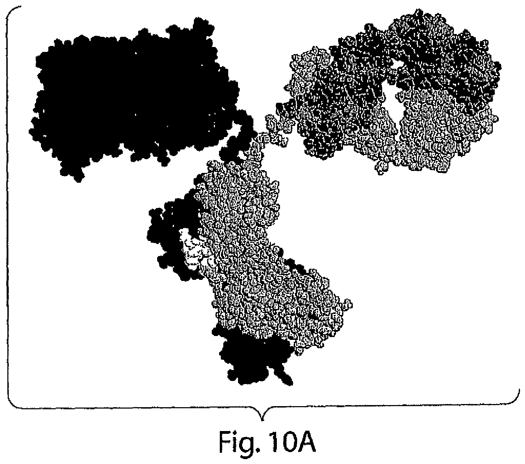

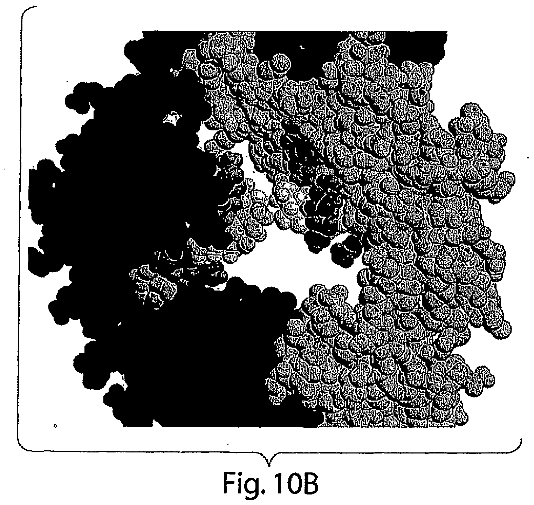

**Fig. 10** illustrates a space filling model of an antibody showing the physical location of the core fucose.

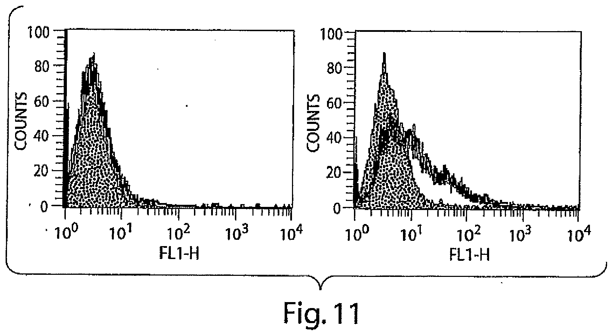

**Fig. 11** demonstrates the binding of antibodies to CHO cells expressing CD137. **Fig. 11A** provides the results for CHO cells without CD137 (negative control); **Fig. 11B** provides the results for CHO cells expressing CD137. The solid curve in every plot is for staining with an irrelevant anti-DNP antibody.

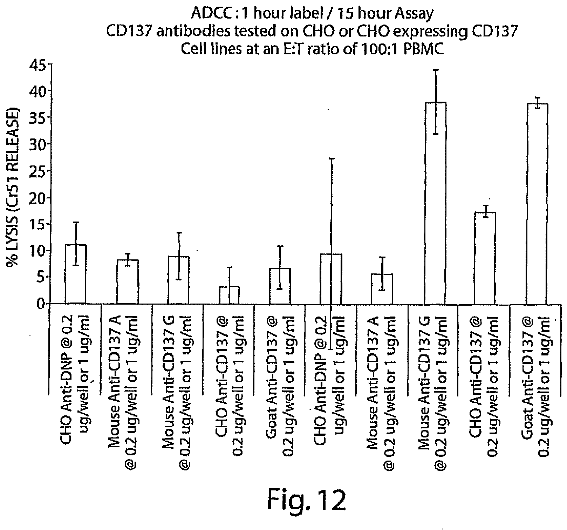

**Fig. 12** illustrates the enhanced killing of CHO cells expressing CD137 by milk-derived antibody in an ADCC assay.

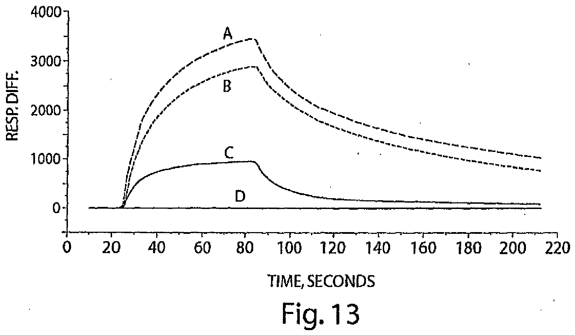

**Fig. 13** provides results from a biosensor analysis of IgG1-sFcgRIIIa binding. **Fig. 13A** shows the results with antibody from goat milk; **Fig. 13B** shows the results with antibody from mouse milk; **Fig. 13C** shows the results with antibody from cell culture; **Fig. 13D** shows the results with aglycosylated antibody from mouse milk.

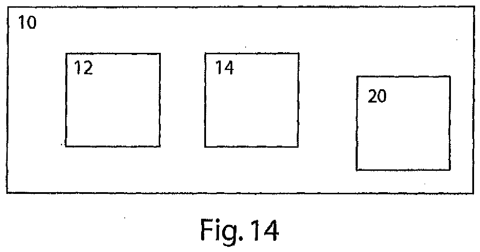

**Fig. 14** illustrates a kit comprising antibody with enhanced ADCC activity.

### DETAILED DESCRIPTION

It has been discovered that antibodies produced in the milk of transgenic animals have enhanced ADCC activity relative to recombinant antibodies produced through *in vitro* cell culture. The glycosylation pattern of antibodies that have enhanced ADCC activity has also been assessed. Compositions of antibodies produced in mammary epithelial cells are provided as are methods of their production and use.

In one aspect of the invention the antibodies have enhanced ADCC activity. As used herein, "enhanced ADCC activity" is intended to refer to an antibody or composition thereof that exhibits greater ADCC activity when produced by a method that alters its glycosylation pattern than when produced by a method that does not alter its glycosylation pattern or than the level of ADCC activity it possesses prior to the alteration of its glycosylation pattern as provided herein. Antibodies with enhanced ADCC activity includes those that prior to the alteration did not exhibit any ADCC activity. As used herein, "glycosylation pattern" refers to the set of carbohydrates that are present on an individual antibody or on a group of antibodies. Changes to the glycosylation pattern can be effected by altering the glycosylation of one antibody or a group of antibodies. Changing the glycosylation pattern of a composition of antibodies is intended to encompass instances where the glycosylation of an individual antibody, a subset of the antibodies in the compositions or all of the antibodies in the composition have been changed. The glycosylation pattern can be determined by many methods known in the art. For example, methods of analyzing carbohydrates on proteins have been described in U.S. Patent Applications published as US 2006-0057638and US 2006-0127950..

Glycosylation is important for the correct folding, targeting, bioactivity and clearance of therapeutic glycoproteins. With the development of, for example, transgenic animals as expression systems, it has been appreciated that different genetic backgrounds result in different expression and glycosylation levels of the produced proteins. The glycosylation of transgenically derived proteins isolated from goat milk is different than that of proteins harvested from cell culture (Denman et al., 1991). In transgenically produced human antithrombin, the type of glycosylation is site-dependent. Fucosylated, sialylated oligosaccharides were found on positions Asn96 and Asn192, whereas Asn155 had an oligomannose oligosaccharide (Edmunds et al., 1998). Site-dependent glycosylation was also found in gamma-interferon produced in mouse milk (James et al., 1995).

Glycosylation is a post-translational modification that can produce a variety of final protein forms in the natural state. IgG molecules are glycosylated at the Asn₂₉₇ residue of the CH2 domain, within the Fc region. Certain alterations in carbohydrate structure also can affect antibody function or therapeutic effectiveness. For instance, in diseases such as rheumatoid arthritis, a higher than normal incidence of agalactosyl structures (which seems to be specific for IgG Fc-associated carbohydrate) has been documented (Parekh et al. 1985; Rademacher et al. 1988a). It has been proposed that this aglycosylated structure is more mobile than the structure normally seen in this region and thus may induce changes in the quaternary structure of the glycoprotein, contribute to the immunogenicity of the antibody, or may itself contribute to aberrant antibody function (Rademacher et al. 1988b; Axford et al. 1992). In the disease state, however, this structure is only one of numerous glycoforms observed.

In some embodiments the antibodies provided herein have at least two-fold higher ADCC activity than the ADCC activity of non-mammary epithelial cell culture-derived antibodies. In other embodiments the glycosylation of the antibodies are modified by the methods provided, and the ADCC activity of the modified antibodies is at least two-fold higher than the ADCC activity of the unmodified version of the same antibody. In some embodiments the antibodies have at least 3-, 4-, 5-, 6-, 7-, 8-, 9-, 10-, 15-, 20-, 25- or 30-fold higher ADCC activity. The antibodies provided are obtained from expression in mammalian mammary epithelial cells. Such antibodies are also referred to herein as mammary epithelial cell-derived antibodies. In some embodiments the antibodies are obtained from expression in the milk of a non-human transgenic animal. Such antibodies are also referred to herein as "milk-derived antibodies". As used herein, "transgenic" refers a cell that includes a nucleic acid molecule that has been inserted by artifice into a cell, or an ancestor thereof, and becomes part of the genome of the animal which develops from that cell. The term is also used in reference to an animal that has had a nucleic acid molecule that has been inserted by artifice into its cells or genome.

Generally, in some embodiments the glycosylation exhibits a high mannose glycosylation pattern. As used herein, a "high mannose glycosylation pattern" is intended to refer to an antibody that contains at least one oligomannose or a composition of antibodies wherein at least 30% of the antibodies contain at least one oligomannose. In some embodiments at least 30%, 40%, 50%, 60%, 70%, 80%, 90% or more of the carbohydrates of the antibodies are oligomannose. In some embodiments at least 30%, 40%, 50%, 60%, 70%, 80%, 90% or more of the carbohydrates of the antibodies are non-fucosylated oligomannose. In other embodiments less than 50%, 40%, 30%, 20%, 10%, 5% or fewer of the carbohydrates of the antibodies are fucose-containing. In still other embodiments the antibodies are low in fucose and high in oligomannose. Therefore, in further embodiments at least 30%, 40%, 50%, 60%, 70%, 80% or 90% or more of the carbohydrates of the antibodies are oligomannose and less than 50%, 40%, 30%, 20%, 10% or 5% of the carbohydrates of the antibodies are fucose-containing. Therefore, in yet a further embodiment at least 30%, 40%, 50%, 60%, 70%, 80% or 90% or more of the carbohydrates of the antibodies are non-fucosylated oligomannose and less than 50%, 40%, 30%, 20%, 10% or 5% of the carbohydrates of the antibodies are fucose-containing. One embodiment of the invention pertains to antibodies with increased ADCC activity that do not have a 1,6-fucose sugar on the heavy chain. In one embodiment, the binding of the Fc region of the antibodies to the FcγRIII receptor found on monocytes, macrophages and natural killer cells, is enhanced if the antibody lacks the core fucose found attached to the Asn297 glycosylation site in the Fc region (e.g., a fucose linked to the base of the bi-antennary structure).

Other antibodies with specific glycosylation patterns are described herein in the **Examples.** As used herein, when compositions of antibodies are discussed, the compositions can be homogeneous or heterogeneous in glycosylation.

The antibodies may be selected for the ability to bind receptors or other proteins, such as those involved in a disease process and/or ADCC. The antibodies can be directed to any cell marker in which providing an antibody directed thereto and having ADCC activity would provide a therapeutic benefit. The antibodies include those that can bind target antigens, such as tumor cell markers. Examples of cell markers and exemplary diseases contemplated herein include CD3, CD4, CD5, CD8, CD14, CD15, CD16, CD19, CD20, CD21, CD22, CD23, CD25, CD32B, CD30, CD33, CD37, CD38, CD40, CD40L, CD46, CD52, CD54, CD59, CD74, CD80, CD126, CD138, CD137 or GPIIbIIIa. In some embodiments the antibodies can be directed to HM1.24, HLA-DR, MUC1, tenascin, PIGF, VEGF, an oncogene, an oncogene product, a necrosis antigen, 17-A1 antigen, IL-2, T101, TAC, IL-6, TRAIL-R1, GD3 ganglioside or TRAIL-R2. Other targets include: anti-CD3 antibodies (e.g., non-Hodgkin's Lymphoma; autoimmune disease - SLE), anti-CD16 antibodies (e.g., FcRIII), anti-CD19 (e.g., non-Hodgkin's Lymphoma), anti-CD20, anti-CD32B antibodies (e.g., FcRIIB and allergy), anti-CD30 (e.g., Hodgkin's Disease), anti-GPIIbIIIa (e.g., Thrombosis), anti-TNF- α (e.g., for rheumatoid arthritis and Crohn's Disease), anti-TEM antibodies for tumor endothelial markers, (e.g., for the control of angiogenesis - anticancer). Once bound to tumor cell markers the antibodies of the invention can recruit monocytes, macrophages and natural killer cells to attack the tumor cells. Antibodies with enhanced ADCC, therefore, can be considered immune modulators that can be effective in shrinking solid tumors and preventing their recurrence.

Extensive studies demonstrated that the stimulation of CD137 by its natural ligand or by agonistic antibodies potentiated an anti-tumor response that resulted in regression of established mouse tumors in various models. CD137 (also called 4-1BB) is a membrane glycoprotein that is expressed in several types of lymphoid cells. An agonistic monoclonal antibody against murine CD 137 has been reported to shrink mouse tumors *in vivo* and prevent their recurrence. Stimulation of CD137 through its natural ligand or agonistic antibodies potentiates the antitumor immune response *in vivo* through stimulation of tumor-reactive effector T-cells and enhanced regulatory NK activity. Systemic administration of the anti-murine CD137 monoclonal antibodies induced complete regression of large tumors in mice such as the poorly immunogenic AGF104A sarcoma and the highly tumorigenic P815 mastocytoma, as well as EL4 thymoma, K1735 melanoma, B10.2 and 87 sarcoma, RENCA renal carcinoma, J558 plasmacytoma, MCA205 sarcoma, JC breast cancer, MCA26 colon cancer and GL261 glioma, alone or in combination with other therapeutic modalities.

The anti-CD 137 antibody in one embodiment of the invention can be cloned and expressed in the milk of several lines of transgenic mice and goats as a genomic "mini-gene." The expression of this gene is under the control of the goat β-casein regulatory elements. Substantial expression of the antibody variants in both mice and goats has been established. In one embodiment the invention provides an anti-CD 137 antibody with enhanced ADCC activity and therefore increased therapeutic properties. According to the current invention, and utilizing the anti-CD 137 antibody produced by transgenic animals as an exemplar of the invention, a non-human:human chimeric monoclonal antibody (i.e., mouse:human) agonist anti-CD 137, was developed. Humanization of the anti-CD 137 antibody is expected to enhance its use for patients undergoing immunotherapy or for other indications. On the basis of the observed amino acid sequence identity, complementary determining regions (CDRs) of the VL and VH regions were grafted onto the human anti-DNA-associated idiotype immunoglobulin clone. It was observed by competitive ELISA that a recombinant chimeric antibody of the invention exhibited a similar bioactivity profile when compared with the murine monoclonal antibody. The anti-CD137 antibody was effective in mediating both antibody-dependent cellular cytotoxicity and complement-mediated cytotoxicity when assayed. Humanization of the antibody sequences are expected to eliminate any undesired human anti-mouse antibody response, allowing for repeated i.v. administration into humans.

As used herein, the term "antibody" refers to a glycoprotein comprising at least two heavy (H) chains and two light (L) chains inter-connected by disulfide bonds, i.e., covalent heterotetramers comprised of two identical Ig H chains and two identical L chains that are encoded by different genes. Each heavy chain is comprised of a heavy chain variable region (abbreviated herein as HCVR or V_{H}) and a heavy chain constant region. The heavy chain constant region is comprised of three domains, C_{H}1, C_{H}2 and C_{H}3. Each light chain is comprised of a light chain variable region (abbreviated herein as LCVR or V_{L}) and a light chain constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. Formation of a mature functional antibody molecule can be accomplished when two proteins are expressed in stoichiometric quantities and self-assemble with the proper configuration.

The term antibodies are meant to encompass antigen-binding fragments thereof. As used herein, an "antigen-binding fragment" of an antibody refers to one or more portions of an antibody that retain the ability to specifically bind to an antigen. It has been shown that the antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, C_{L} and C_{H}1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546) which consists of a V_{H} domain; and (vi) an isolated complementarity determining region (CDR). Furthermore, although the two domains of the Fv fragment, V and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional procedures, such as proteolytic fragmentation procedures, as described in J. Goding, Monoclonal Antibodies: Principles and Practice, pp 98-118 (N.Y. Academic Press 1983), as well as by other techniques known to those with skill in the art. The fragments are screened for utility in the same manner as are intact antibodies.

Preferred antigen-binding fragments include a Fab fragment, a F(ab')₂ fragment, and a Fv fragment CDR3. In one embodiment the antibody fragment is part of a Fc fusion polypeptide. As an example, the antibody fragment is an Fc region containing N-linked oligosaccharides.

An "isolated antibody", as used herein, is intended to refer to an antibody which is substantially free of other antibodies having different antigenic specificities. An isolated antibody that specifically binds to an epitope, isoform or variant of an antigen may, however, have cross-reactivity to other related antigens, e.g., from other species. Moreover, an isolated antibody may be substantially free of other cellular material and/or chemicals. As used herein, "specific binding" refers to antibody binding to a predetermined antigen. Typically, the antibody binds with an affinity that is at least two-fold greater than its affinity for binding to a non-specific antigen other than the predetermined antigen or a closely-related antigen. Therefore, the antibodies provided herein in some embodiments specifically bind a target antigen.

The antibodies are of the isotype IgG. In further embodiments, the antibodies are selected from the group consisting of IgG1, IgG2, IgG3, IgG4,or has immunoglobulin constant and/or variable domain of IgG1, IgG2, IgG3, IgG4.. In other embodiments, the antibodies are bispecific or multispecific antibodies. In still other embodiments, the antibodies are recombinant antibodies, polyclonal antibodies, monoclonal antibodies, humanized antibodies or chimeric antibodies, or a mixture of these. In some embodiments the chimeric antibody is a genetically engineered fusion of parts of a non-human (e.g., mouse, rat, rabbit) antibody with parts of a human antibody. The chimeric antibodies, in some embodiments, can contain approximately 33% non-human protein and 67% human protein. With specific regard to mouse chimerics, they can be developed to reduce the HAMA response elicited by murine antibodies, as they can combine the specificity of the murine antibody with the efficient human immune system interaction of a human antibody.

In other embodiments, the antibodies are recombinant antibodies. The term "recombinant antibody", as used herein, is intended to include antibodies that are prepared, expressed, created or isolated by recombinant means, such as antibodies isolated from an animal that is transgenic for another species' immunoglobulin genes, antibodies expressed using a recombinant expression vector transfected into a host cell, antibodies isolated from a recombinant, combinatorial antibody library, or antibodies prepared, expressed, created or isolated by any other means that involves splicing of immunoglobulin gene sequences to other DNA sequences.

In yet other embodiments, the antibodies can be chimeric or humanized antibodies. As used herein, the term "chimeric antibody" refers to an antibody, that combines the murine variable or hypervariable regions with the human constant region or constant and variable framework regions. As used herein, the term "humanized antibody" refers to an antibody that retains only the antigen-binding CDRs from the parent antibody in association with human framework regions (see, Waldmann, 1991, Science 252:1657). Such chimeric or humanized antibodies retaining binding specificity of the murine antibody are expected to have reduced immunogenicity when administered *in vivo* for diagnostic, prophylactic or therapeutic applications according to the invention.

In another embodiment of the invention the antibody is a humanized antibody with increased ADCC activity. In one embodiment this antibody is CD137. Humanization (also called Reshaping or CDR-grafting) is an established technique for reducing the immunogenicity of monoclonal antibodies from xenogeneic sources, such as mice. Humanized antibodies can be generated through standard molecular biology techniques. In one embodiment this comprises grafting of the rodent complementarity-determining regions (CDRs) into a human framework. However, this technique is mostly an iterative process and a number of elements come into play when designing a humanized antibody: the length of the CDRs, the human frameworks and the substitution of residues from the rodent mAb into the human framework regions (backmutations).

Therapeutic mouse mAbs are at times not ideal for human use because the HAMA (human anti-mouse antibodies) response neutralizes the antibody, and clears it quickly from the circulation and, in the worst case, induces serious allergic hypersensitivity. Several strategies have been developed to replace most of the murine Ig sequences with human sequences, resulting in fewer side effects while retaining efficacy. One strategy for developing a human therapeutic mAb is to replace the murine heavy chain (H) and light chain (L) constant regions (C_{H} and C_{L}, respectively), or generically non-human chains, with human regions so that the resulting chimeric antibody is comprised mostly of human IgG protein sequence except for the antigen-binding domains that would remain non-human. This strategy was used for the development of Rituxan® (Rituximab anti-human CD20, Genentech), the first monoclonal antibody approved in the U.S., used to treat non-Hodgkin lymphoma. By some estimates, providing therapeutic mAbs with human C_{H} and C_{L} sequences should eliminate approximately 90% of the immunogenicity of murine antibody proteins.

According to one aspect of the invention, a non-human : human chimeric monoclonal antibody (i.e., mouse : human) anti-CD137 antibody, is provided. Complementary determining regions (CDRs) of the V_{L} and V_{H} regions of the mouse antibody were grafted onto the human anti-DNA-associated idiotype immunoglobulin clone. By competitive ELISA, the recombinant chimeric antibody of the invention exhibited a similar bioactivity profile when compared with the murine monoclonal antibody. The anti-CD137 antibody was effective in mediating both antibody-dependent cellular cytotoxicity (ADCC) and complement-mediated cytotoxicity.

According to an alternative embodiment, the monoclonal antibodies of the present invention can be modified to be in the form of a bispecific antibody, or a multispecific antibody. The term "bispecific antibody" is intended to include any agent, e.g., a protein, peptide, or protein or peptide complex, which has two different binding specificities which bind to, or interact with (a) a cell surface antigen and (b) an Fc receptor on the surface of an effector cell. The term "multispecific antibody" is intended to include any agent, e.g., a protein, peptide, or protein or peptide complex, which has more than two different binding specificities which bind to, or interact with (a) a cell surface antigen, (b) an Fc receptor on the surface of an effector cell, and (c) at least one other component. Accordingly, the invention includes, but is not limited to, bispecific, trispecific, tetraspecific, and other multispecific antibodies which are directed to cell surface antigens, and to Fc receptors on effector cells. The term "bispecific antibodies" further includes diabodies. Diabodies are bivalent, bispecific antibodies in which the V_{H} and V_{L} domains are expressed on a single polypeptide chain, but using a linker that is too short to allow for pairing between the two domains on the same chain, thereby forcing the domains to pair with complementary domains of another chain and creating two antigen-binding sites (see e.g., Holliger, P., et al. (1993) Proc. Natl. Acad. Sci. USA 90:6444-6448; Poijak, R.J., et al. (1994) Structure 2:1121-1123).

In certain embodiments, the antibodies are human antibodies. The term "human antibody", as used herein, is intended to include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo).* However, the term "human antibody", as used herein, is not intended to include antibodies in which CDR sequences derived from the germline of another mammalian species, such as a mouse have been grafted onto human framework sequences (referred to herein as "humanized antibodies"). Human antibodies are generated using transgenic mice carrying parts of the human immune system rather than the mouse system.

Fully human monoclonal antibodies also can be prepared by immunizing mice transgenic for large portions of human immunoglobulin heavy and light chain loci. See, e.g., U.S. patents 5,591,669, 5,598,369, 5,545,806, 5,545,807, 6,150,584, and references cited therein. These animals have been genetically modified such that there is a functional deletion in the production of endogenous (e.g., murine) antibodies. The animals are further modified to contain all or a portion of the human germ-line immunoglobulin gene locus such that immunization of these animals results in the production of fully human antibodies to the antigen of interest. Following immunization of these mice (e.g., XenoMouse (Abgenix), HuMAb mice (Medarex/GenPharm)), monoclonal antibodies are prepared according to standard hybridoma technology. These monoclonal antibodies have human immunoglobulin amino acid sequences and therefore will not provoke human anti-mouse antibody (HAMA) responses when administered to humans. The human antibodies, like any of the antibodies provided herein can be monoclonal antibodies, polyclonal antibodies or a mixture of monoclonal and polyclonal antibodies.

In some embodiments the antibody is a full-length antibody. In some embodiments the full-length antibody comprises a heavy chain and a light chain.

Procedures for raising polyclonal antibodies are well known. For example antibodies are raised by administering an antigen subcutaneously to New Zealand white rabbits which have first been bled to obtain pre-immune serum. The antigen can be injected at a total volume of 100 µl per site at six different sites, typically with one or more adjustments. The rabbits are then bled two weeks after the first injection and periodically boosted with the same antigen three times every six weeks. A sample of serum is collected 10 days after each boost. Polyclonal antibodies are recovered from the serum, preferably by affinity chromatography using antigen to capture the antibody. This and other procedures for raising polyclonal antibodies are disclosed in E. Harlow, et. al., editors, Antibodies: A Laboratory Manual (1988).

To produce monoclonal antibodies, mice are injected multiple times (see above), the mice spleens are removed and resuspended in a phosphate buffered saline (PBS). The spleen cells serve as a source of lymphocytes, some of which are producing antibody of the appropriate specificity. These are then fused with a permanently growing myeloma partner cell, and the products of the fusion are plated into a number of tissue culture wells in the presence of a selective agent such as HAT. The wells are then screened to identify those containing cells making useful antibody by ELISA. These are then freshly plated. After a period of growth, these wells are again screened to identify antibody-producing cells. Several cloning procedures are carried out until over 90% of the wells contain single clones which are positive for antibody production. From this procedure a stable line of clones is established which produce the antibody. The monoclonal antibody can then be purified by affinity chromatography using Protein A Sepharose, ion-exchange chromatography, as well as variations and combinations of these techniques (See e.g. US Patent 6,998,467).

An alternative strategy for developing a mAb product is to produce the antibody in transgenic mice in which the entire native Ig repertoire has been replaced with human Ig genes. Such mice produce fully human antibody proteins. In this way a chimeric, humanized or fully human antibody is produced, both embodiments of the current invention. Both antibodies will have an effector function and are useful in the treatment of cancer and cancerous lesions. The chimeric antibody embodiment of the current invention retains the original murine variable (antigen-binding) sequences and hence should retain its binding and functional properties.

The antibody can have a glycosylation pattern as provided anywhere herein, which provides or enhances the ADCC activity of the antibody. The invention embraces all methods of generating antibodies with increased ADCC activity as provided herein. In one embodiment the methods comprise generating fully human monoclonal antibodies. In another embodiment the methods comprises the use of transgenic mice, or other non-human animal subject, that have their antibody genes replaced by human genes and that produce 'humanized' antibodies in response to immunization with an antigen of interest. In yet another embodiment phage displays can be used to engineer bacteriophages to display a fully human monoclonal antibody on their surface. Antibodies generated by any of the above methods can be used to generate transgenic animals. In one embodiment this is done by integrating a DNA construct encoding the antibody into the genome of the transgenic animal.

Milk produced in transgenic animals, including mice and goats, can comprise large amounts of oligomannose-containing antibody. These oligomannose containing antibodies have an enhanced ADCC profile and function as a preferred target for target receptors of monocytes, macrophages and natural killers cells. Methods are provided for recombinantly producing the antibodies described herein so that they are produced in mammalian mammary epithelial cells. This can be accomplished in cell culture. This can also be accomplished via expression of the antibodies in the milk of transgenic animals.

In one embodiment the mammalian mammary epithelial cells have been engineered to express the antibody in the milk of the transgenic animal, such as a mouse or goat. The expression of this gene is, for example, under the control of the goat β-casein regulatory elements. Substantial expression of the antibodies in both mice and goats has been established. The transgenic animals can be generated by co-transfecting separate constructs containing the H and L chains, or one construct containing both chains. In certain embodiments, both transgenes integrate into the same chromosomal site so that the genes are transmitted together to progeny and protein expression is jointly regulated. In some embodiments the expression is optimized for individual mammary duct epithelial cells that produce milk proteins. The animals can be, for example, dairy animals, such as goats and cattle, or mice.

For example, to produce primary cell lines containing a construct (e.g., encoding a chimeric anti-human CD 137) for use in producing transgenic goats by nuclear transfer, the heavy and light chain constructs can be transfected into primary goat skin epithelial cells, which are clonally expanded and fully characterized to assess transgene copy number, transgene structural integrity and chromosomal integration site. As used herein, "nuclear transfer" refers to a method of cloning wherein the nucleus from a donor cell is transplanted into an enucleated oocyte.

Coding sequences for proteins of interest can be obtained by screening libraries of genomic material or reverse-translated messenger RNA derived from the animal of choice (such as cattle or mice), obtained from sequence databases such as NCBI, Genbank, or by obtaining the sequences of antibodies, etc. The sequences can be cloned into an appropriate plasmid vector and amplified in a suitable host organism, like *E. coli.* After amplification of the vector, the DNA construct can be excised, purified from the remains of the vector and introduced into expression vectors that can be used to produce transgenic animals. The transgenic animals will have the desired transgenic protein integrated into their genome.

After amplification of the vector, the DNA construct could be excised with the appropriate 5' and 3' control sequences, purified away from the remains of the vector and used to produce transgenic animals that have integrated into their genome the desired non-glycosylated related transgenic protein. Conversely, with some vectors, such as yeast artificial chromosomes (YACs), it is not necessary to remove the assembled construct from the vector; in such cases the amplified vector may be used directly to make transgenic animals. The coding sequence can be operatively linked to a control sequence which enables the coding sequence to be expressed in the milk of a transgenic non-human mammal.

A DNA sequence which is suitable for directing production to the milk of transgenic animals can carry a 5'-promoter region derived from a naturally-derived milk protein. This promoter is consequently under the control of hormonal and tissue-specific factors and is most active in lactating mammary tissue. In some embodiments the promoter is a caprine beta casein promoter. The promoter can be operably linked to a DNA sequence directing the production of a protein leader sequence which directs the secretion of the transgenic protein across the mammary epithelium into the milk. In some embodiments a 3'-sequence, which can be derived from a naturally secreted milk protein, can be added to improve stability of mRNA.

As used herein, a "leader sequence" or "signal sequence" is a nucleic acid sequence that encodes a protein secretory signal, and, when operably linked to a downstream nucleic acid molecule encoding a transgenic protein directs secretion. The leader sequence may be the native human leader sequence, an artificially-derived leader, or may obtained from the same gene as the promoter used to direct transcription of the transgene coding sequence, or from another protein that is normally secreted from a cell, such as a mammalian mammary epithelial cell.

In some embodiments the promoters are milk-specific promoters. As used herein, a "milk-specific promoter" is a promoter that naturally directs expression of a gene in a cell that secretes a protein into milk (e.g., a mammary epithelial cell) and includes, for example, the casein promoters, e.g., α-casein promoter (e.g., alpha S-1 casein promoter and alpha S2-casein promoter), β-casein promoter (e.g., the goat beta casein gene promoter (DiTullio, BIOTECHNOLOGY 10:74-77, 1992), γ-casein promoter, κ-casein promoter, whey acidic protein (WAP) promoter (Gorton et al., BIOTECHNOLOGY 5: 1183-1187, 1987), β-lactoglobulin promoter (Clark et al., BIOTECHNOLOGY 7: 487-492, 1989) and α-lactalbumin promoter (Soulier et al., FEBS LETTS. 297:13, 1992). Also included in this definition are promoters that are specifically activated in mammary tissue, such as, for example, the long terminal repeat (LTR) promoter of the mouse mammary tumor virus (MMTV).

As used herein, a coding sequence and regulatory sequences are said to be "operably joined" when they are covalently linked in such a way as to place the expression or transcription of the coding sequence under the influence or control of the regulatory sequences. In order that the coding sequences be translated into a functional protein the coding sequences are operably joined to regulatory sequences. Two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids and phagemids. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium, or just a single time per host as the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., ß-galactosidase or alkaline phosphatase), and genes which visibly affect the phenotype of transformed or transfected cells, hosts, colonies or plaques. Preferred vectors are those capable of autonomous replication and expression of the structural gene products present in the DNA segments to which they are operably joined.

It is described a method for the production of a transgenic antibody, and variants and fragments thereof, the process comprises expressing in the milk of a transgenic non-human mammal a transgenic antibody encoded by a nucleic acid construct. In one embodiment the method for producing the antibodies of the invention comprises:
(a) transfecting non-human mammalian cells with a transgene DNA construct encoding a desired transgenic antibody;
(b) selecting cells in which said transgene DNA construct has been inserted into the genome of the cells; and
(c) performing a first nuclear transfer procedure to generate a non-human transgenic mammal heterozygous for the desired transgenic antibody and that can express it in its milk.

In another aspect the method comprises:
(a) providing a non-human transgenic mammal engineered to express an antibody,
(b) expressing the antibody in the milk of the non-human transgenic mammal; and
(c) isolating the antibodies expressed in the milk.

Such methods can further comprise steps for inducing lactation as well as steps for determining the ADCC activity of the antibodies obtained. The methods can also further comprise additional isolation and/or purification steps. The methods can also comprise steps for comparing the ADCC activity of the antibodies obtained with antibodies produced in cell culture. The ADCC activity of the antibodies obtained can, in some embodiments, be compared to antibodies produced by non-mammary epithelial cells. Such cells can be cells of a cell culture. Experimental techniques for assessing the ADCC activity of the antibodies can be any of those known to those of ordinary skill in the art or as provided herein, such as below in the Examples. The antibodies can be obtained, in some embodiments, by collecting the antibodies from the milk of a transgenic animal produced as provided herein or from an offspring of said transgenic animal.

In some embodiments the construct encoding the desired antibody (or antibody fusion polypeptide) is actuated by at least one beta-casein promoter. In other embodiments the non-human transgenic mammal is an ungulate. In still other embodiments the non-human transgenic mammal is a goat.

In some embodiments the antibodies produced by the transgenic mammal is produced at a level of at least 1 gram per liter of milk produced.

Transgenic animals, capable of recombinant antibody expression, can also be generated according to methods known in the art (See e.g., U.S. Patent No. 5,945,577). Animals suitable for transgenic expression, include, but are not limited to goat, sheep, bison, camel, cow, pig, rabbit, buffalo, horse, rat, mouse or llama. Suitable animals also include bovine, caprine, ovine and porcine, which relate to various species of cows, goats, sheep and pigs (or swine), respectively. Suitable animals also include ungulates. As used herein, "ungulate" is of or relating to a hoofed typically herbivorous quadruped mammal, including, without limitation, sheep, swine, goats, cattle and horses. In one embodiment, the animals are generated by co-transfecting primary cells with separate constructs containing the heavy and light chains. These cells are then used for nuclear transfer. Alternatively, if microinjection was used to generate the transgenic animals, the constructs are co-injected. Should alterations need to be made to glycosylation levels in an embodiment site-directed mutagenesis can be used.

Cloning will result in a multiplicity of transgenic animals - each capable of producing an antibody or other gene construct of interest. The production methods include the use of the cloned animals and the offspring of those animals. In some embodiments the cloned animals are caprines, bovines or mice. Cloning also encompasses the nuclear transfer of fetuses, nuclear transfer, tissue and organ transplantation and the creation of chimeric offspring.

One step of the cloning process comprises transferring the genome of a cell that contains the transgene of interest into an enucleated oocyte. As used herein, "transgene" refers to any piece of a nucleic acid molecule that is inserted by artifice into a cell, or an ancestor thereof, and becomes part of the genome of an animal which develops from that cell. Such a transgene may include a gene which is partly or entirely exogenous (i.e., foreign) to the transgenic animal, or may represent a gene having identity to an endogenous gene of the animal.

Suitable mammalian sources for oocytes include goats, sheep, cows, pigs, rabbits, guinea pigs, mice, hamsters, rats, non-human primates, etc. Preferably, oocytes are obtained from ungulates, and most preferably goats or cattle. Methods for isolation of oocytes are well known in the art. Essentially, the process comprises isolating oocytes from the ovaries or reproductive tract of a mammal, e.g., a goat. A readily available source of ungulate oocytes is from hormonally-induced female animals. For the successful use of techniques such as genetic engineering, nuclear transfer and cloning, oocytes may preferably be matured *in vivo* before these cells may be used as recipient cells for nuclear transfer, and before they were fertilized by the sperm cell to develop into an embryo. Metaphase II stage oocytes, which have been matured *in vivo,* have been successfully used in nuclear transfer techniques. Essentially, mature metaphase II oocytes are collected surgically from either non-super ovulated or super ovulated animals several hours past the onset of estrus or past the injection of human chorionic gonadotropin (hCG) or similar hormone.

One of the tools used to predict the quantity and quality of the recombinant protein expressed in the mammary gland is through the induction of lactation (Ebert KM, 1994). Induced lactation allows for the expression and analysis of protein from the early stage of transgenic production rather than from the first natural lactation resulting from pregnancy, which is at least a year later. Induction of lactation can be done either hormonally or manually. It is possible that various lactation procedures, especially hormonally-induced lactation, might affect the transcriptional regulation of glycosyltransferases in mammary glands. N-linked oligosaccharides from various lactation samples of cloned animals were similar except for the content of NeuGc. Carbohydrates in transgenic antibody production from natural lactation contained higher amounts of NeuGc than that from other lactation procedures, even though the overall sialic acid content in samples from different lactation was comparable. Likewise, it appears that transgenic proteins produced in the milk of goats are also comprised of a complex mixture of individual protein species (Zhou, 2005).

Protein A-purified IgG fractions isolated from pooled milk samples from each line were analyzed *in vitro* to characterize antibody binding specificity and affinity and dose-dependent enhancement of T-cell proliferation. In one embodiment, milk-derived glycosylated and aglycosylated chimeric preparations to the original GW mAb.

Production of healthy transgenic mice with normal growth and reproductive characteristics and reasonable levels (> 1 mg/ml) of bioactive antibody was established. Production in mice with a given construct can be a precursor to work in large-scale production in species such as caprines or bovines. The production and characterization of chimeric anti-CD 137 led to testing of one or more of these preparations in a mouse model to demonstrate anti-tumor activity *in vivo.* Both the glycosylated and aglycosylated chimeric antibody constructs thereafter were used to generate transgenic goats to express anti-CD137 in their milk.

The ability to modify animal genomes through transgenic technology offers new alternatives for the manufacture of recombinant proteins with modified glycosylation patterns. The production of human recombinant pharmaceuticals in the milk of transgenic farm animals solves many of the problems associated with microbial bioreactors (e.g., lack of post-translational modifications, improper protein folding, high purification costs) or animal cell bioreactors (e.g., high capital costs, expensive culture media, low yields). The current invention in some embodiments includes the use of transgenic production of antibodies in the milk of transgenic animals homozygous for a desired gene that optimizes the glycosylation pattern of those molecules.

According to another embodiment, the glycosylation pattern of a target molecule can be, for example, modified through alterations in the feed to a non-human mammal (Kerr et al., 2003).

It is further described methods for producing an antibody comprising collecting the antibody from the milk of a transgenic non-human mammal engineered to express the antibody in its milk, and determining the ADCC activity of the antibody. In some embodiments the ADCC activity is compared to the ADCC activity of antibodies expressed in cell culture. In another embodiment antibody is collected from mammary epithelial cells in culture engineered to express the antibody, and the ADCC activity of the antibody is determined. In some embodiments the ADCC activity is compared to the ADCC activity of antibodies expressed in cell culture. In some embodiments the cells of the cell culture are non-mammary epithelial cells. Assays for assessing ADCC activity are provided below in the **Examples** and are also known in the art.

As used herein, the term "substantially pure" means that the proteins are essentially free of other substances to an extent practical and appropriate for their intended use. In particular, the proteins are sufficiently pure and are sufficiently free from other biological constituents of their hosts cells so as to be useful in, for example, protein sequencing, or producing pharmaceutical preparations. In some embodiments, therefore, the antibodies are substantially pure.

As used herein with respect to polypeptides, "isolated" means separated from its native environment and present in sufficient quantity to permit its identification or use. Isolated, when referring to a protein or polypeptide, means, for example: (i) selectively produced by expression cloning or (ii) purified as by chromatography or electrophoresis. Isolated proteins or polypeptides may be, but need not be, substantially pure. Because an isolated polypeptide may be admixed with a pharmaceutically acceptable carrier in a pharmaceutical preparation, the polypeptide may comprise only a small percentage by weight of the preparation. The polypeptide is nonetheless isolated in that it has been separated from the substances with which it may be associated in living systems, i.e., isolated from other proteins. In some embodiments, therefore, the antibodies are isolated.

The antibodies exhibit enhanced ADCC activity, which is an important factor in the successful use of monoclonal antibodies in therapy. Simultaneous binding of the constant or Fc region of the antibodies of the invention can induce a biological response. One of these responses is the induction of cellular apoptosis in the target cell or ADCC. ADCC functions through the binding of the Fc region to the Fc gamma receptors, which are located on the surface of monocytes, macrophages and natural killer cells. Upon binding to the receptor, these cells are activated and release cytokines and oxidative free radicals that can kill the target cell (the cell that contained the antigen target).

Therefore, in one aspect, it is described antibody preparations with increased ADCC activity and therefore enhanced therapeutic properties. The compositions provided can be used to treat a subject in which ADCC activity would confer at least some medical benefit. The composition provided, therefore, can be used to treat a subject with a disease. In some embodiments the antibodies can be used to treat a subject by enhancing ADCC activity in a subject through administration of the antibody.

The term "treating", "treat" or "treatment" as used herein includes preventative (e.g., prophylactic) and palliative treatment.

Specific indications against which the compositions provided could provide beneficial therapeutic effects include cancer, such as an effective immunomodulatory treatment of solid tumors, melanomas; as well as carcinomas of the breast, colon, ovaries, kidney, prostate and lung.

"Cancer" as used herein refers to an uncontrolled growth of cells which interferes with the normal functioning of the bodily organs and systems. Cancers which migrate from their original location and seed vital organs can eventually lead to the death of the subject through the functional deterioration of the affected organs. Cancers include Hodgkin's lymphoma, non-Hodgkin's lymphoma, mycosis fungoides/Sezary syndrome, histiocytosis X, chronic lymphocytic leukaemia, hairy cell leukaemia, multiple myeloma, Waldenstrom's macroglobulinaemia, cryoglobulinaemia and heavy chain disease. Hemopoietic cancers, such as leukemia, are able to outcompete the normal hemopoietic compartments in a subject, thereby leading to hemopoietic failure (in the form of anemia, thrombocytopenia and neutropenia) ultimately causing death.

A metastasis is a region of cancer cells, distinct from the primary tumor location resulting from the dissemination of cancer cells from the primary tumor to other parts of the body. At the time of diagnosis of the primary tumor mass, the subject may be monitored for the presence of metastases. Metastases are most often detected through the sole or combined use of magnetic resonance imaging (MRI) scans, computed tomography (CT) scans, blood and platelet counts, liver function studies, chest X-rays and bone scans in addition to the monitoring of specific symptoms.

Cancer, as used herein, includes the following types of cancer, breast cancer, biliary tract cancer; bladder cancer; brain cancer including glioblastomas and medulloblastomas; cervical cancer; choriocarcinoma; colon cancer; endometrial cancer; esophageal cancer; gastric cancer; leukemia; hematological neoplasms including acute lymphocytic and myelogenous leukemia; T-cell acute lymphoblastic leukemia/lymphoma; hairy cell leukemia; chromic myelogenous leukemia, multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Other cancers will be known to one of ordinary skill in the art and include mastocytoma, thymoma, plasmacytoma and glioma.

The compositions of the invention are also useful for treating immune disorders. An "immune disorder" includes adult respiratory distress syndrome, arteriosclerosis, asthma, atherosclerosis, cholecystitis, cirrhosis, Crohn's disease, diabetes mellitus, emphysema, hypereosinophilia, inflammation, irritable bowel syndrome, multiple sclerosis, myasthenia gravis, myocardial or pericardial inflammation, osteoarthritis, osteoporosis, pancreatitis, rheumatoid arthritis, scleroderma, and colitis (see, e.g., US published application 2003/0175754).

The compositions described herein are also useful for treating autoimmune disease, which include but are not limited to systemic lupus erythematosus, lupus nephritis, diabetes mellitus, inflammatory bowel disease, celiac disease, an autoimmune thyroid disease, Addison's disease, Sjogren's syndrome, Sydenham's chorea, Takayasu's arteritis, Wegener's granulomatosis, autoimmune gastritis, autoimmune hepatitis, cutaneous autoimmune diseases, autoimmune dilated cardiomyopathy, multiple sclerosis, myocarditis, myasthenia gravis, pernicious anemia, polymyalgia, psoriasis, rapidly progressive glomerulonephritis, rheumatoid arthritis, ulcerative colitis, vasculitis, autoimmune diseases of the muscle, autoimmune diseases of the testis, autoimmune diseases of the ovary and autoimmune diseases of the eye.

In another embodiment antibodies with increased ADCC activity are effective in the treatment of autoimmune derived encephalo-myelitis, systemic lupus erythematosis, as well as other disease states.

The antibodies provided herein can be combined with other therapeutic agents. The antibodies and other therapeutic agent may be administered simultaneously or sequentially. When the other therapeutic agents are administered simultaneously they can be administered in the same or separate formulations, but are administered at the same time. The other therapeutic agents are administered sequentially with one another and with the antibodies, when the administration of the other therapeutic agents and the antibodies is temporally separated. The separation in time between the administration of these compounds may be a matter of minutes or it may be longer.

Other therapeutic agents include, for example, but are not limited to anti cancer therapies. Anti-cancer therapies include cancer medicaments, radiation and surgical procedures. As used herein, a "cancer medicament" refers to an agent which is administered to a subject for the purpose of treating a cancer. As used herein, "treating cancer" includes preventing the development of a cancer, reducing the symptoms of cancer, and/or inhibiting the growth of an established cancer. In other aspects, the cancer medicament is administered to a subject at risk of developing a cancer for the purpose of reducing the risk of developing the cancer. Various types of medicaments for the treatment of cancer are described herein. For the purpose of this specification, cancer medicaments are classified as chemotherapeutic agents, immunotherapeutic agents, cancer vaccines, hormone therapy and biological response modifiers.

The chemotherapeutic agent may be selected from the group consisting of methotrexate, vincristine, adriamycin, cisplatin, non-sugar containing chloroethylnitrosoureas, 5-fluorouracil, mitomycin C, bleomycin, doxorubicin, dacarbazine, taxol, fragyline, Meglamine GLA, valrubicin, carmustaine and poliferposan, MMI270, BAY 12-9566, RAS famesyl transferase inhibitor, famesyl transferase inhibitor, MMP, MTA/LY231514, LY264618/Lometexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Paclitaxel, Taxol/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara/Fludarabine, Pharmarubicin/Epirubicin, DepoCyt, ZD1839, LU 79553/Bis-Naphtalimide, LU 103793/Dolastain, Caetyx/liposomal doxorubicin, Gemzar/Gemcitabine, ZD 0473/Anormed, YM 116, lodine seeds, CDK4 and CDK2 inhibitors, PARP inhibitors, D4809/Dexifosamide, Ifes/Mesnex/Ifosamide, Vumon/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, Cytarabine HCI, Dactinomycin, Daunorubicin HCl, Estramustine phosphate sodium, Etoposide (VP16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analogue), Lomustine (CCNU), Mechlorethamine HCl (nitrogen mustard), Mercaptopurine, Mesna, Mitotane (o.p'-DDD), Mitoxantrone HCl, Octreotide, Plicamycin, Procarbazine HCl, Streptozocin, Tamoxifen citrate, Thioguanine, Thiotepa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26) and Vindesine sulfate, but it is not so limited.

The immunotherapeutic agent may be selected from the group consisting of Ributaxin, Herceptin, Quadramet, Panorex, IDEC-Y2B8, BEC2, C225, Oncolym, SMART M195, ATRAGEN, Ovarex, Bexxar, LDP-03, ior t6, MDX-210, MDX-11, MDX-22, OV103, 3622W94, anti-VEGF, Zenapax, MDX-220, MDX-447, MELIMMUNE-2, MELIMMUNE-1, CEACIDE, Pretarget, NovoMAb-G2, TNT, Gliomab-H, GNI-250, EMD-72000, LymphoCide, CMA 676, Monopharm-C, 4B5, ior egf.r3, ior c5, BABS, anti-FLK-2, MDX-260, ANA Ab, SMART 1D10 Ab, SMART ABL 364 Ab and ImmuRAIT-CEA, but it is not so limited.

The cancer vaccine may be selected from the group consisting of EGF, Anti-idiotypic cancer vaccines, Gp75 antigen, GMK melanoma vaccine, MGV ganglioside conjugate vaccine, Her2/neu, Ovarex, M-Vax, O-Vax, L-Vax, STn-KHL theratope, BLP25 (MUC-1), liposomal idiotypic vaccine, Melacine, peptide antigen vaccines, toxin/antigen vaccines, MVA-based vaccine, PACIS, BCG vaccine, TA-HPV, TA-CIN, DISC-virus and ImmuCyst/TheraCys, but it is not so limited.

The additional therapeutic agents can also be immunomodulators. Examples of immunomodulators that can be administered are IL-1, IL-2, IL-3, IL-6, IL-10, IL-12, IL-18, IL-21, an interferon, paclitaxel, TNF-α or a combination thereof, but are not so limited. The antibodies of the invention can also be administered in combination with immunomodulators and/or additional anti-cancer agents, such as IL-21, IL-12, and/or trastuzumab.

The compositions provided are useful in effective amounts. The term effective amount refers to the amount necessary or sufficient to realize a desired biologic effect. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is effective to treat the particular subject. The effective amount for any particular application can vary depending on such factors as the disease or condition being treated, the particular composition being administered, the size of the subject, or the severity of the disease or condition. One of ordinary skill in the art can empirically determine the effective amount of a particular composition without necessitating undue experimentation. It is preferred generally that a maximum dose be used, that is, the highest safe dose according to some medical judgment. Multiple doses per day may be contemplated to achieve appropriate systemic levels of compounds. Appropriate system levels can be determined by, for example, measurement of the patient's peak or sustained plasma level of the drug. "Dose" and "dosage" are used interchangeably herein.

Determining a therapeutically effective amount specifically depends on such factors as toxicity and efficacy of the medicament. Toxicity may be determined using methods well known in the art. Efficacy may be determined utilizing the same guidance. A pharmaceutically effective amount, therefore, is an amount that is deemed by the clinician to be toxicologically tolerable, yet efficacious. Efficacy, for example, can be measured by the induction or substantial induction of T-lymphocyte cytotoxicity at the targeted tissue or a decrease in mass of the targeted tissue. According to a preferred embodiment suitable dosages are expected to be from about 1 mg/kg to 10 mg/kg.

According to embodiments that involve administering to a subject in need of treatment a therapeutically effective amount of the antibodies as provided herein, "therapeutically effective" denotes the amount of antibody needed to inhibit or reverse a disease condition (e.g., reduce or inhibit cancer growth). Some methods contemplate combination therapy with known cancer medicaments or therapies, for example, chemotherapy (preferably using compounds of the sort listed herein) or radiation. The patient may be a human or non-human animal. A patient typically is in need of treatment when suffering from a cancer characterized by increased levels of receptors that promote cancer maintenance or proliferation.

Generally, daily oral doses of active compounds will be from about 0.01 milligrams/kg per day to 1000 milligrams/kg per day. It is expected that oral doses in the range of 0.5 to 50 milligrams/kg, in one or several administrations per day, will yield the desired results. Dosage may be adjusted appropriately to achieve desired drug levels, local or systemic, depending upon the mode of administration. For example, it is expected that intravenous administration would be from an order to several orders of magnitude lower dose per day. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of antibodies.

In some embodiments the compositions provided are employed for *in vivo* applications. Depending on the intended mode of administration *in vivo* the compositions used may be in the dosage form of solid, semi-solid or liquid such as, e.g., tablets, pills, powders, capsules, gels, ointments, liquids, suspensions, or the like. Preferably the compositions are administered in unit dosage forms suitable for single administration of precise dosage amounts. The compositions may also include, depending on the formulation desired, pharmaceutically acceptable carriers or diluents, which are defined as aqueous-based vehicles commonly used to formulate pharmaceutical compositions for animal or human administration. The diluent is selected so as not to affect the biological activity of the human recombinant protein of interest. Examples of such diluents are distilled water, physiological saline, Ringer's solution, dextrose solution, and Hank's solution. The same diluents may be used to reconstitute lyophilized a human recombinant protein of interest. In addition, the pharmaceutical composition may also include other medicinal agents, pharmaceutical agents, carriers, adjuvants, nontoxic, non-therapeutic, non-immunogenic stabilizers, etc. Effective amounts of such diluent or carrier are amounts which are effective to obtain a pharmaceutically acceptable formulation in terms of solubility of components, biological activity, etc. In some embodiments the compositions provided herein are sterile.

The compositions herein may be administered to human patients via oral, parenteral or topical administrations and otherwise systemic forms for anti-melanoma, anti-lymphoma, anti-leukemia and anti-breast cancer treatment. The compositions can also be utilized therapeutically for a range of autoimmune disorders, such as rheumatoid arthritis, systemic lupis, multiple sclerosis, etc.

Administration during *in vivo* treatment may be by any number of routes, including parenteral and oral, but preferably parenteral. Intracapsular, intravenous, intrathecal, and intraperitoneal routes of administration may be employed, generally intravenous is preferred. The skilled artisan recognizes that the route of administration varies depending on the disorder to be treated.

For use in therapy, an effective amount of the compositions can be administered to a subject by any mode that delivers the composition to the desired surface. Administering the pharmaceutical composition descibed herein may be accomplished by any means known to the skilled artisan. Preferred routes of administration include but are not limited to oral, parenteral, intramuscular, intranasal, sublingual, intratracheal, inhalation, ocular, vaginal, and rectal.

The compounds, when it is desirable to deliver them systemically, may be formulated for parenteral administration by injection, *e.g*., by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, *e.g*., sterile pyrogen-free water, before use.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the composition may take the form of tablets or lozenges formulated in conventional manner.

For oral administration, for example, the antibodies can be formulated readily by combining the active antibodies with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl- cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, i.e. EDTA for neutralizing internal acid conditions or may be administered without any carriers.

Also specifically contemplated are oral dosage forms of the antibodies. The component or components may be chemically modified so that oral delivery of the antibodies is efficacious. Generally, the chemical modification contemplated is the attachment of at least one moiety to the antibodies, where said moiety permits (a) inhibition of proteolysis; and (b) uptake into the blood stream from the stomach or intestine. Also desired is the increase in overall stability of the antibodies and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, 1981, "Soluble Polymer-Enzyme Adducts" In: Enzymes as Drugs, Hocenberg and Roberts, eds., Wiley-Interscience, New York, NY, pp. 367-383; Newmark, et al., 1982, J. Appl. Biochem. 4:185-189. Other polymers that could be used are poly-1,3-dioxolane and poly-1,3,6-tioxocane. Preferred for pharmaceutical usage, as indicated above, are polyethylene glycol moieties.

For the compositions the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the antibody or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the compositions may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, a-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential non-ionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation either alone or as a mixture in different ratios.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the compounds for use according to the present invention may be conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.* gelatin for use in an inhaler or insufflator may be formulated containing a powder mix of the compound and a suitable powder base such as lactose or starch.

Also contemplated herein is pulmonary delivery. The compositions can be delivered to the lungs of a mammal while inhaling and traverses across the lung epithelial lining to the blood stream. Other reports of inhaled molecules include Adjei et al., 1990, Pharmaceutical Research, 7:565-569; Adjei et al., 1990, International Journal of Pharmaceutics, 63:135-144 (leuprolide acetate); Braquet et al., 1989, Journal of Cardiovascular Pharmacology, 13(suppl. 5):143-146 (endothelin-1); Hubbard et al., 1989, Annals of Internal Medicine, Vol. III, pp. 206-212 (a1- antitrypsin); Smith et al., 1989, J. Clin. Invest. 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J. Immunol. 140:3482-3488 (interferon-g and tumor necrosis factor alpha) and Platz et al., U.S. Patent No. 5,284,656 (granulocyte colony stimulating factor). A method and composition for pulmonary delivery of drugs for systemic effect is described in U.S. Patent No. 5,451,569, issued September 19, 1995 to Wong et al.

A wide range of mechanical devices designed for pulmonary delivery of therapeutic products may be used, including but not limited to nebulizers, metered dose inhalers, and powder inhalers, all of which are familiar to those skilled in the art.

Some specific examples of commercially available suitable devices are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts.

All such devices require the use of formulations suitable for dispensing. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified antibodies may also be prepared in different formulations depending on the type of chemical modification or the type of device employed.

Formulations suitable for use with a nebulizer, either jet or ultrasonic, will typically comprise the therapeutic dissolved in water at a concentration of about 0.1 to 25 mg of biologically active therapeutic per mL of solution. The formulation may also include a buffer and a simple sugar *(e.g.,* for antibody stabilization and regulation of osmotic pressure). The nebulizer formulation may also contain a surfactant, to reduce or prevent surface induced aggregation of the therapeutic caused by atomization of the solution in forming the aerosol.

Formulations for use with a metered-dose inhaler device will generally comprise a finely divided powder containing the therapeutic suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing the therapeutic and may also include a bulking agent, such as lactose, sorbitol, sucrose, or mannitol in amounts which facilitate dispersal of the powder from the device, *e.g.,* 50 to 90% by weight of the formulation. The therapeutic can be prepared in particulate form with an average particle size of less than 10 mm (or microns), most preferably 0.5 to 5 mm, for most effective delivery to the distal lung.

Nasal delivery of a pharmaceutical composition described herein is also contemplated. Nasal delivery allows the passage of a pharmaceutical composition of the present invention to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the pharmaceutical composition of the present invention solution into a chamber of defined volume, which chamber has an aperture dimensioned to aerosolize and aerosol formulation by forming a spray when a liquid in the chamber is compressed. The chamber is compressed to administer the pharmaceutical composition of the present invention. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The compounds may also be formulated in rectal or vaginal compositions such as suppositories or retention enemas, *e.g*., containing conventional suppository bases such as cocoa butter or other glycerides.

In addition to the formulations described previously, the compounds may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer, Science 249:1527-1533, 1990.

The antibodies and optionally other therapeutics may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions described herein contain an effective amount of the antibodies and optionally therapeutic agents included in a pharmaceutically-acceptable carrier. The term pharmaceutically-acceptable carrier means one or more compatible solid or liquid filler, diluents or encapsulating substances which are suitable for administration to a human or other vertebrate animal. The term carrier denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The therapeutic agent(s), including specifically but not limited to the antibodies, may be provided in particles. Particles as used herein means nano or microparticles (or in some instances larger) which can consist in whole or in part of the antibody. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero order release, first order release, second order release, delayed release, sustained release, immediate release, and any combination thereof, etc. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the antibody in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bioerodible hydrogels described by H.S. Sawhney, C.P. Pathak and J.A. Hubell in Macromolecules, (1993) 26:581-587, the teachings of which are incorporated herein. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The therapeutic agent(s) may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug there from. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30-60 days. Long-term sustained release implants are well-known to those of ordinary skill in the art and include some of the release systems described above.

Also provided herein are kits containing the antibodies provided. Fig. 14 shows an example of such a kit. The kit 10 includes an antibody 12. The kit 10 may also contain one or more vials or containers 14. The kit also includes instructions for administering the component(s) to a subject who has a disease described herein, such as cancer, or who has symptoms of such a disease.

In some embodiments, the kit 10 can include a pharmaceutical preparation vial, a pharmaceutical preparation diluent vial, and the antibodies. The vial containing the diluent for the pharmaceutical preparation is optional. The diluent vial contains a diluent such as physiological saline for diluting what could be a concentrated solution or lyophilized powder of the antibody. The instructions can include instructions for mixing a particular amount of the diluent with a particular amount of the concentrated pharmaceutical preparation, whereby a final formulation for injection or infusion is prepared. The instructions may include instructions for use in a syringe or other adminstration device. The instructions 20 can include instructions for treating a patient with an effective amount of the antibodies. It also will be understood that the containers containing the preparations, whether the container is a bottle, a vial with a septum, an ampoule with a septum, an infusion bag, and the like, can contain indicia such as conventional markings which change color when the preparation has been autoclaved or otherwise sterilized.

A "subject" shall mean a human or vertebrate mammal including but not limited to a dog, cat, horse, cow, pig, sheep, goat, or primate, e.g., monkey.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well-known in the art. Generally, nomenclatures used in connection with, and techniques of biochemistry, enzymology, molecular and cellular biology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated.

The present invention is further illustrated by the following Examples.

### EXAMPLES

### Materials and Methods

### Sequencing of mRNA from Hybridoma

RNA was prepared with a Qiagen RNeasy Mini kit (Cat # 74104). On the 4th day, 13 ml of culture was centrifuged for 5 minutes and resuspended in PBS. It was centrifuged again for 5 min. The pellet was resuspended in 600 µl of RNeasy RLT containing 6 µl of µ-ME. The lysate was passed through a 22g needle 5 times and 600 µl of 70 % EtOH was added and mixed. Seven hundred ul aliquots were applied to the RNeasy column twice, centrifuged 30 seconds, and washed with 700 µl of RW1. It was washed twice with 500 ul of PPE, dried 1 minute, and eluted with 50 µl of water twice.

Two µl of the RNA was reverse transcribed with the Promega Reverse Transcription System (Cat # A3500) using oligodT primers. The reaction was incubated at 42°C for 1 hour and then heated to 95°C for 5 minutes. The reaction was then diluted to 100 µl with water. PCR was carried out on 1 µl aliquots of cDNA using primers chosen from one for the N terminus or 5' end and one for the C terminus or 3' end. PCR was carried out using primers only from the constant region as a positive control.

PCR products were purified with the Qiagen QiaQuick PCR Purification Kit (Cat #28104). An additional elution was done to make the final volume 100 µl. The absorbance at 260 nm was measured. Concentrations varied from 10-26 ng/µl and 100 ng was given in for sequencing along with the N terminus primer used for the PCR.

Since the amino terminal primers used were part of the coding sequence of the amino terminus of the antibody, they could introduce mutations into the sequence. Using the sequences obtained, the germline genes were identified from the mouse genome. Then primers were synthesized to termini of these genes for the PCR of the entire coding sequence from the cDNA. In this way the entire coding region of the antibody were obtained free of any sequences contributed by PCR primers. The coding sequence is the sequence of the expressed antibody since it is consistent with the amino terminus sequence in each case. The J regions were identified from the known J regions as in annotated in the sequence.

### Cloning and Sequencing the Heavy and Light Chain Genes for Anti-Human CD137

Transgene expression vectors containing human constant region sequences for the four major IgG subclasses were constructed. These vectors carry the goat beta-casein promoter and other 5' and 3' regulatory sequences that ensure mammary-specific transgene expression. The chimeric antibody variant was constructed by inserting the variable region sequences of a mouse anti-human CD137 antibody. The anti-CD 137 H and L chains of the murine sequences were identified by sequencing the corresponding variable region. A collection of oligonucleotides that represent sequences from the 5' coding region of various families of murine immunoglobulins were assembled. Sequences of murine immunoglobulins were used individually as 5' primers to amplify cDNA prepared from hybridoma RNA, and the resulting PCR products were cloned and sequenced. The 3' PCR primers were prepared from the known sequences of the constant regions. The PCR primers included restriction endonuclease sites so that the resulting amplified sequences could be inserted into expression vectors. These sequences were inserted into the constructs to produce genes encoding chimeric proteins.

The methods used to clone and sequencing the anti-CD137 antibody gene variable regions included the following steps 1)-5). 1) cDNA was made from hybridoma RNA. RNA were prepared from the hybridoma by standard methods, and cDNA were prepared by reverse transcription with a commercially available kit of reagents (Reverse Transcription System, Promega, Madison, WI). 2) cDNA was amplified by PCR with primers based on known sequences from the amino terminus of the V_{H} and V_{L} regions from several well-characterized murine monoclonal antibodies. 3) The variable region sequences were amplified by inserting the PCR-generated sequences into cloning vectors with the neomycin resistance (neo^{R}) selectable marker, and neo^{R} colonies were isolated. 4) H and L chain cDNA prepared from approximately 6 colonies were sequenced to determine the consensus sequence for each variable region. It was important to ensure that no mutations had been introduced into the sequences from PCR artifacts. DNA sequencing was performed on a fee-for-service basis by SequeGen, Co. (Worcester, MA). 5) The H and L proteins isolated from the hybridoma supernatant were sequenced, and the actual protein sequences were compared to the deduced protein sequences derived from the gene sequences. This step confirmed that the cloned genes encode functional antibody chains. Protein sequencing was performed on a fee-for-service basis by Cardinal Health (San Diego, CA).

### The Production of Separate Chimeric IgG Heavy and Light Chain Constructs for Chimeric Antibodies

Variable region sequences obtained according to the methods provided above were used to replace human variable region sequences in an existing human IgG1 antibody. In one embodiment of the invention human variable region sequences in IgG1 expression vectors were used along with murine variable region sequences to produce a chimeric humanized antibody. The antibody expression vectors contained the IgG1 H gene in its native glycosylable form. The IgG1 glycosylation site is an Asn residue at position 297 in the CH2 domain. An aglycosylated form of the IgG1 H chain was generated by altering Asn₂₉₇ to Gln₂₉₇ by site-specific mutagenesis in the gene sequence. This provided three constructs: L chain, glycosylated H chain and aglycosylated H chain. Two forms of each construct were used for testing. Each construct was evaluated by restriction mapping and Southern blot analysis and used for the generation of transgenic animals. In addition, the constructs were used in transient transfection studies to test bioactivity of the genetically engineered chimeric protein.

The constructs used for transgenic animal development contained the goat β-casein promoter and other 5' and 3' regulatory sequences that are used to ensure high level mammary-specific transgene expression. Because of the cross-species recognition of the promoter and other regulatory elements, the same construct was used to generate transgenic mice and goats. Once bred, the goats and/or mice were screened for glycosylation levels and enhanced ADCC properties. The structural integrity of the constructs by restriction mapping was confirmed. The constructs were used to make transiently transfected cells and transgenic animals.

### Construction of Heavy Chain Chimera and Insertion into Expression Vector

In order to construct the heavy chain chimera whereby the mouse IgG2a constant region was replaced by the human IgG1 constant region, the BC2083 expression vector containing the human antibody sequences with a mouse leader sequence was used (Plasmid 1). A splice donor site was eliminated by a G to A silent mutation which did not change the coding for glycine near the C terminus. Unique sites were put into the BC2083 expression vector surrounding the variable region. DraIII and PmlI were put into the N terminus and ApaI exists in the amino portion of the heavy constant region. These sites were cloned into the XhoI sites of BC2083 by PCR of the zeo gene from CMV-Zeo with the primers to give p80 BC2083 zeo (Plasmid 2). This rapid method of restriction site insertion into plasmids utilizes the zeocin resistance conferred by the zeo gene. Zeocin resistance was selected for by using 25 µg/ml of zeocin in NZYCM agar.

The human IgG1 constant portion was put back into the unique ApaI and XhoI sites by cutting it out of BC2083 and cloning it into p80 to give p83 BC2083 DraIII IgG1 (Plasmid 3). This plasmid has unique DraIII/PmlI and ApaI sites flanking the heavy variable region so that any heavy variable region was attached to the human IgG1 constant region coding sequences.

The heavy chain variable region of the anti-CD137 antibody was prepared for insertion by putting DraIII and PmlI sites on the amino terminus and an Apa site on the C terminus by PCR. The ApaI site is naturally occurring near the amino terminus of the human IgG1 constant region. PCR was performed with primers MHE and MHEC using PfuTurbo (Stratagene Cat No. 600153-81) and cDNA. The PCR fragment was cloned into pCR-BluntII-TOPO (Invitrogen Cat. No.: K28602) and sequenced with primers pcr2.1f and pcr2.1b. This gave p96, containing the heavy chain variable region flanked by DraIII-PmlI and ApaI (Plasmid 4).

The beta-casein expression vector, p100 BC2083 heavy (BC2197) (Plasmid 5), was constructed by isolating the p96 pCR-BluntII-Mayo-heavy DraIII-ApaI fragment and ligating it to cut DraIII-ApaI cut p83 BC2083 DraIII IgG1 (Plasmid 3).

To block the glycosylation by changing the target asparagine to a glutamine, the heavy chain coding sequence was prepared by PCR with PfuTurbo from BC2083 using primers heavy constant N and heavy constant C subcloned into pCR-Zero -Blunt. This gave p76 and p77 pCR2.1-Blunt-IgG1-heavy-constant. These plasmids were sequenced to ensure that no mutations were introduced into the constant region during the PCR. According to an embodiment, the subcloned constant region of the anti-CD 137 antibody in p77 was mutagenized using the QuickChange XL Mutagenesis (Stratagene) kit and the mutagenic oligos. This oligo changes asparagine 297 to a glutamine and removes a nearby BsaAI site to facilitate screening by restriction enzyme analysis by the silent mutation of a threonine codon. This gave plasmids p88, p89 and p90 pCR2.1-Blunt-IgG1-heavy-mut. PCR was carried out on these plasmids with the primers to prepare a fragment for sequencing.

### Construction of Light Chain Chimera and Insertion Into Expression Vector

The expression vector used for the light chain was BC1060 (Plasmid 6). To enable the fusion of the variable region to the human kappa constant region, two restriction sites were engineered into the mouse J region in order. A KpnI site was introduced by changing the codon for a glycine from GGG or GGC to GGT. The coding sequence for a leucine was changed to CTT from CTG to create a HindIII site (Plasmid 8).

Using PCR with PfuTurbo (Stratagene), the coding region of the human constant region of the kappa chain was isolated from BC1060 with KpnI and HindIII sites at the beginning and a naturally occurring SacI site near the end of the coding region using primers. The PCR product was cloned into ZERO Blunt TOPO PCR W EC (Invitrogen Cat. No.: K286020). These plasmids were sequenced. This made p85 pcr-blunt-1060 kappa constant rev (Plasmid 7) and p86 pcr-blunt-1060 kappa constant (Plasmid 8).

Similarly, the variable region was isolated from cDNA by PCR with primers and cloned into pCR2.1-Blunt-TOPO to make p92 pCR2.1-Blunt- kappa variable (Plasmid 9) where the variable region is flanked by a XhoI site at nucleotide 340 and KpnI and HindIIII sites around nucleotide 731. These plasmids were sequenced.

The light chain chimera was first constructed in pCR-Blunt using 3 pieces of DNA. The backbone from XhoI to SacI was supplied by p86 pcr-blunt-1060 kappa constant. The kappa constant region was the HindIII-SacI piece from p85 pcr-blunt-1060 kappa constant rev. The variable region was supplied by p92 pCR-Blunt- kappa variable rev using the XhoI-HindIII piece. Colonies were checked by PCR with primers, pcr2.1f and pcr2.1b, by looking for production of a 863 bp fragment. This gives p94 pCR-BluntII-Mayo-kap-chim (Plasmid 10). The plasmid was checked by cutting with XhoI and SacI to give a 684 bp fragment.

The light chain chimera was put into the beta-casein expression vector BC1060 containing the Immunogen human light chain with the mouse heavy leader sequence. p94 was cut with XhoI-SacI, and the small piece isolated. BC1060 was cut with KpnI-SacI, and the 5206 bp piece was isolated. BC1060 was cut with KpnI, XhoI and PacI to isolate the large backbone. These three pieces were ligated, and colonies were screened with the needed primers. The positive plasmid was checked with BglII, and the PCR product was sequenced. This plasmid is p104 BC1060 LC chim (BC2198) (Plasmid 11).

### Construction of Cell Culture Expression Vectors

To construct the transient expression vector for the light chain, the XhoI fragment from p104 BC1060 LC chim (Plasmid 11) was ligated into the XhoI site of pCEP4 to give p106 and p107 pCEP4-Mayo-LC (#2203) (Plasmid 12). Positive colonies were detected by PCR with oligos CEPF and KVC.

To construct the transient expression vector for the heavy chain, the BamHI fragment of p100 BC2083 heavy chain was cloned into BamHI cut pCEP4. Colonies were screened by PCR with HVC 09 and CEPF. This resulted in plasmid p110 pCEP4-BamHI-HC (#2202) (Plasmid 13).

The chimera with the heavy chain variable region of the anti-CD 137 antibody was prepared by ligating the small KpnI-AgeI piece of #110 pCEP4-BamHI-HC (#2202) containing the variable region into KpnI-AgeI cut #88 pCR2.1-Blunt-IgG1-heavy-mut. This gives plasmid p111 pCR2.1-Mayo-IgG1-heavy-mut (Plasmid 14). This plasmid was checked with BsaAI-PstI.

To construct the transient expression vector, the small XhoI fragment from p111 containing the chimeric antibody coding region was inserted into the XhoI site of pCEP4. Colonies were checked by PCR with HVC C09 and CEPF. This gave p112 pCEP4-Xho-Mayo-IgG1-aglycos (BC2206). Expected fragments were obtained with EcoRV-HindIII digestion (2479 bp) and BamHI digestion (1454 bp). To construct the beta casein expression vector, the small XhoI fragment from p111 containing the chimeric antibody coding region was inserted into the XhoI site of BC2083. Colonies were checked by PCR with oligos HVC 09 and CA5. Digestion with MluI-Eco47III-NotI gave the expected 2479 bp fragment, while digestion with BamHI gave the expected 1454 bp fragment.

### Cloning an IgG1 Mutant

The anti-CD137 antibody used was expressed in mouse milk. For mouse expression, the construction techniques for BC2197 (p100 BC2083 heavy) and (BC2198) p104 BC1060 light chain were used. Likewise, the parental plasmids were BC2083 for the heavy chain or BC 1060 for the light chain. Basically, the variable regions including the leader sequences in the parental plasmids were exchanged with the cDNA sequence from the variable region of the heavy and light chains of the anti-CD 137 antibody cDNA. The constant regions of the expression vector was replaced, IgG1 of the heavy chain and kappa of the light chain, with sequences which were cloned.

### Cloning of IgG1 Sequences

The heavy chain of the antibody utilized was cloned from a cDNA purchased from Invitrogen. PCR with PfuTurbo was performed using placental cDNA and the primers shown below. The C terminus primer 61960C11 has a base change with respect to the wild type sequence to destroy a splice donor site. The 993 bp fragment was cloned into ZeroBlunt. The sequences, indicated that one sequence was of the G1m(3). There are inherited differences associated with gamma-globulin from human serum (Grubb 1956; Grubb and Laurell 1956). There is a similar system for Km (Kappa marker, previously referred as Inv (or Inv) which stands for ëInhibitrice Virmí). This is the Caucasian allotype G1m(f) or G1m(3) instead of the African allotype G1m(z) or G1m(17) as found in the starting plasmid.

In order to clone the other allotype, PCR was done from a brain cDNA as above to give plasmids, p116, p 117, p118 and p 119. None of these plasmids had the correct sequence. For example, most of the plasmids were missing the ApaI and/or the XhoI sites at the end of the sequence, which should have been provided by the PCR primers. The PCR was done again using p116 as template or brain cDNA.

PCR of p116 yielded 121, 122 and 123. PCR of brain cDNA yielded 124. The insert from plasmid 121 was used to make p133, p134, p135 and p136, which are BC2083 heavy G1m(17) by cutting 100c BC2083 heavy with ApaI and XhoI and p121 ZeroBlunt-IgG1 G1m(17) with ApaI and XhoI, ligating and selecting on kanamycin. p133 was used.

For the mouse expression, only the variable regions was changed in plasmid BC2083, an expression vector containing the human antibody sequences with a mouse leader sequence. This has a splice donor site at the end of the IgG1 constant region eliminated by a G to A silent mutation which did not change the coding for glycine. In an effort to enhance goat expression, the constant region was changed to an IgG1 constant region that was subsequently cloned. The cloned constant region from p114 was used to create p137 and 138. p100 BC2083 heavy (BC2197) was cut with ApaI-XhoI and 114 ZeroBlunt-IgG1 G1m(3) cut with ApaI and XhoI and ligated to give p138 (BC2228). The cloned constant region from p121 (G1m(17)) was used to create p133, 134, 135 and 136 BC2083 heavy G1m(17). The kappa constant region was also replaced with one cloned by GTC Biotherapeutics. SEQ. ID. NO. 1: AGGGTACCAAGCTTGAAATCAAACGAAC-Kappa Constant Human H01; SEQ. ID. NO. 2: 5'AAGGGTCCGGATCCTCGAGGATCCTAACACTCTCCCCTGTTGAAGCTC-Human Kappa C #7734.

### Production of Skin Fibroblast Lines

Fibroblasts from fresh goat skin biopsy samples were maintained in primary culture *in vitro.* Briefly, skin samples were minced in Ca⁺⁺-free and Mg⁺⁺-free phosphate buffered saline (PBS), harvested with dilute trypsin in EDTA to recover single cell suspensions and cultured at 37°C. Confluent cells were trypsinized and sub-cultured. Aliquots of cells were cryopreserved in liquid nitrogen for future use.

### Analysis of Transfected Cell Lines

Transfected cells were characterized by Southern blot analysis with probes specific for the transgenes, such as beta-casein, chimeric anti-CD137 H and L chain cDNAs, to establish the transgene copy number and identify potential rearrangements. Each cell line also was analyzed by FISH to confirm single integration and to determine chromosomal location. Cytogenetic analysis was performed to confirm the karyotypes of the cell lines.

### FISH

For Interphase FISH, a few hundred cells from each expanded colony were immobilized on filters and hybridized to amplified transgene-specific digoxigenin-labeled probes. For metaphase FISH, cells were cultured on Lab Tek Chamber slides (Nunc, Rochester, NY) and pulsed with 5-bromo-2'deoxyuridine (BrdU) to allow for replication banding. Probe binding was detected with FITC-conjugated anti-digoxigenin, and the chromosomes were counterstained with 4',6-Diamidino-2-phenylindole (DAPI). Images were captured using a Zeiss Axioskop microscope (Zeiss Imaging, Thornwood, NY), a Hamamatsu digital camera (Hamamatsu, Bridgewater, NJ), and Image Pro-Plus software (Media Cybernetics, Silver Springs, MD). Most probes were relatively large and easy to detect. Probes for individual IgG H and L chains, which were encoded by relatively short cDNA sequences, were too small to give good resolution by themselves. These small probes were mixed with sequences from the milk-specific promoter for goat beta-casein.

### Cytogenetic Analysis

Cytogenetic analysis of donor transfected fibroblast cell lines was carried out. Transgene probes were labeled with digoxigenin-dUTP by nick translation. Probe binding to the denatured chromosomes were detected either with FITC-conjugated anti-digoxigenin or with horseradish peroxidase-conjugated anti-digoxigenin followed by FITC-conjugated tyramide. Chromosome banding patterns were visualized with DAPI. Goats have 60 chromosomes, all of them acrocentric (having the centromere at one end rather than at or near the middle). The metaphase spreads were inspected for evidence of gross abnormalities such as chromosome loss, duplication or gross rearrangement. Cell lines that were used to generate first-generation transgenic goats were karyo-typically normal and carried structurally intact chimeric anti-CD137 H and L chain genes along with the beta-casein promoter and other essential regulatory elements.

### Generation of Transgenic Animals Expressing Chimeric Anti-Human CD137 in Milk

Glycosylated and non-glycosylated versions of chimeric anti-human CD137 antibodies have been produced. After the purification of sufficient quantities of antibody from milk to test the bioactivity it was found that though they were essentially produced at identical levels, the activity profiles of the two forms differed.

Transgene constructs for the chimeric antibodies were used to generate transgenic mice and goats. Transgenic animals produce mature antibodies by introducing a 1:1 mixture of H chain and L chain constructs. The L chain construct was combined with either the glycosylated or aglycosylated H chain construct. Specifically, the relative and absolute levels of bioactive product in milk was measure by Western blot analysis and antibody binding measurement *in vitro.*

### Transgenic Mice

A practical strategy for testing the feasibility of the inducible systems in transgenic mice was to evaluate transgenic protein expression in the milk of first-generation (F₁) mice. It has been determined that in some transgenic animals, the original transgene constructs integrate into chromosomal sites in cell divisions following microinjection. The founder animals are, therefore, chimeric which can affect the levels of expression of the transgene. These chromosomal integration sites will segregate in the following generation to form stable, homogeneous transgenic animal lines. Therefore, F₁ mice are reasonable models for determining the stability of transgene expression and the biological products that they produce. Moreover, in order for mice to lactate, they must mature (which takes about 2 months), mate and produce offspring. After analysis it was determined that the secretion levels were stable and the construct used was effective.

Linear DNA from each construct prepared was purified by CsCl gradient followed by electroelution, and transgenic mice were generated by pronuclear microinjection. Transgenic founder animals were identified by PCR analysis of tail tissue DNA, and relative copy number was determined using Southern blot analysis. A number of transgenic first-generation transgene-bearing "founder" (F₀) females were produced for each construct (glycosylated and aglycosylated). These F₀ mice were mated at maturity to initiate lactation. Their milk was analyzed on Western blots developed with goat anti-human-Fc antibody to identify mice that secrete structurally intact chimeric antibodies bearing the human C_{H} region.

The best founders, defined as healthy animals with the maximum reasonable expression of antibody in their milk, were then bred to generate F1 females, which were used to provide sufficient milk to be collected for antibody testing *in vitro* and *in vivo.*

### Transgenic Goats

Transgenic goats with pre-defined genetics were generated using nuclear transfer techniques routine in the art. Nuclear transfer eliminates the problem with transgene mosaicism in the first few generations because all of the animals derived from a transgenic cell line should be fully transgenic. The transgene construct was introduced into primary cell lines by a standard transfection method, like lipofection or electroporation. The recombinant primary cell lines were screened *in vitro* for important characteristics, such as transgene copy-number, integrity and integration site, before they were used to produce transgenic animals. Female goat skin fibroblasts were used to make the transfected transgenic cells that served as nuclear donors for nuclear transfer, resulting in an all female offspring. Milk containing the recombinant protein could, therefore, be obtained directly from F₀ goats. The relative and absolute levels of bioactive product in milk was measured by Western blot analysis.

### Results

### Purification And Characterization of Antibodies

Four different chimeric anti-CD137 antibodies were generated. One variant was aglycosylated by mutation of Asn297 to a glutamine. This antibody variant can function as a negative control in functional studies as aglycosylated IgGs are known not to bind to Fc receptors or to be active in ADCC (Nose et al., 1983). Two other antibody variants were prepared from milk, one from mouse milk and one from goat milk. The fourth antibody variant was expressed in HEK 293 cells, a human cell line.

One of the antibody variants was expressed in human cells. The human embryonic kidney 293 cell line (293) is suitable for transient transfection technology as it can be efficiently transfected. A genetic variant stably expressing the EBV EBNA1 protein (293E), which provides significantly higher protein expression when EBV's oriP is present in the vector backbone, was used.

The increased expression obtained in oriP/EBNA1 systems appears to be independent of episomal replication when performing transient transfection, as removal of the DS domain of oriP, which is responsible for initiation of DNA replication in EBNA1 positive cells does not reduce transgene expression, while removal of FR but not DS strongly reduces expression. The increased expression is thus likely due to the combined effect of the EBNA1- dependent enhancer activity of oriP and to the increased nuclear import of plasmids, owing to the presence of a nuclear localization signal in EBNA1 (Pham et al., 2003).

pCEP4 (Cat # V04450; Invitrogen, Carlsbad, CA), a vector designed for high-level constitutive expression from the CMV promoter, was used to express the antibody. The 293EBNA/ebv vector host system represents a significant improvement over COS7/SV40ori based systems (Jalanko et al., 1988; Shen et al., 1995). An important issue for high level recombinant protein expression is to use vectors with promoters that are highly active in the host cell line, such as the CMV promoter, which is particularly powerful in 293 cells, because it is transactivated by the constitutively expressed adenovirus E1a protein. (Durocher et al., 2002).

A hybridoma (GW) producing a monoclonal antibody specific for human CD137 was generated by fusing the spleen B cells of a BALB/c mouse that had been immunized with a fusion protein of the human CD137 extracellular portion and the mouse IgG2a Fc region to a mouse plasmacytoma by standard methods as previously described (Wilcox et al., 2002, J Clin Inv 109: 651-659). The anti-CD137 antibodies are chimeric versions of a mouse monoclonal antibody (mAb) to human CD137 where the constant regions have been replaced in the heavy chain and light chain by the human IgG1 constant region and the human kappa constant region, respectively. This antibody was expressed in mouse and goat milk using a beta casein promoter (Pollock et al., J Immunol Meth 231: 147-157) or in HEK 293 cells using a cytomegalovirus (CMV) promoter by transient transfection. Goat milk was from hormonally induced lactations of transgenic goats (Ebert et al., 1994, Biotech 12: 699-702). The antibodies were purified by Protein A chromatography. Goat milk antibody was purified after 9 days of lactation to avoid goat antibody that contaminates the colostrum from earlier milkings. Similar preparations of goat milk-derived chimeric anti-CD137 antibody are 90% monomer and 10% aggregates by size-exclusion chromatography. Antibody concentrations were measured by absorbance at 280 nm, and using the value of 1.4 mg/ml, is 1 OD as the conversion factor.

### Sugar Analysis

Asparagine-linked oligosaccharides were released using PNGase F at 37°C overnight in 50 mM sodium phosphate buffer, pH 7.0, containing 1% beta-mercaptoethanol. The samples were analyzed by matrix-assisted laser desorption-ionization time-of-flight (MALDI-TOF) mass spectrometry (MS) analysis using a Voyager-DE PRO Biospectrometry Workstation (Applied Biosystems, Foster City, CA, USA). MALDI-TOF MS analysis was performed with a 2,5-dihydroxybenzoic acid/2-hydroxy-5-methoxybenzoic acid (9:1, v/v) matrix in positive ion, reflective mode. The major carbohydrate (63%) in mouse milk-derived antibody is non-fucosylated Man5. **Fig. 1** shows a MALDI-TOF MS analysis of the N-glycans that were released from the antibodies by PNGase F digestion. Comparing the results from the mouse milk-derived antibody with the results from the cell culture-derived antibody, it is clear that the major oligosaccharide from the mouse milk-derived antibody is Man5 which contains 5 mannose residues and 2 GlcNAc residues.

High-pressure liquid chromatography (HPLC) analysis was done on 2-aminobenzoic acid-labeled oligosaccharides released by PNGase F after overnight digestion at 37°C. Eighty micrograms of antibody were treated with PNGFase overnight at 37°C. The samples were filtered through a 10-kDa filter and then dialyzed against water overnight in a Biodialyzer. The sample was dried down, labeled with 2-aminobenzoic acid and cleaned up to remove excess label. After evaporation to dryness, the sample was reconstituted with 500 µl of water and 100 µl injected onto an Asahipak NH2P-50 4D column (4.6 X 250 mm, Phenomenex) using an HP1100 system equipped with a fluorescent detector (230 nm as excitation and 425 nm as emission), according to a method described by Anumula et al. (Cammusco et al., 2000, Anim Biotech 11; 1-17). Man5 and Man6 standards were purchased from Prozyme. Quantitation was done by HPLC analysis and is shown in **Fig. 2****.** Confirming that the species from the mouse-milk derived antibody, which runs identically to standard Man5, is a mannose oligonucleotide was the demonstration of its Endo H sensitivity (Maley et al., 1981 and Tarentino et al., 1974). After treatment with Endo H, the Man5 peak disappears, confirming the identity of the peak seen at 36.5 minutes in the mouse milk-derived sample of antibody (**Figs. 2** and **3**). There are minor species present such as core fucose-containing G1F (16%) and G2F (21%), as well as lesser amounts of non-fucosylated G1 and Man6. Chicken IgG, which contains oligomannose structures, has species at 2068 and 1906, corresponding to Man8 and Glc1Man8 (Raju et al.). Although Man6 is detectable at 1420, higher mannose oligosaccharides at 1582, 1744 and 1906 are clearly missing. In contrast, the major species present in HEK 293 cell derived material are fucosylated G0F (55%) and G1F (37%), with G2F as a minor species (8%). HEK 293 cells are known to inefficiently sialylate proteins made at high levels (Chitlaru et al, 2002; Chitlaru et al., 1998).

Both **Fig. 1**(MS-data) and **Fig. 2** show that the carbohydrate compositions of milk-derived antibody and cell culture-derived antibody are significantly different. **Fig. 2** is a comparison of oligosaccharide maps obtained by HPLC with fluorescence detection and shows that the major carbohydrate (63%) in mouse milk-derived antibody is non-fucosylated Man5, while the major carbohydrates of cell culture-derived antibody are fucosylated. Aglycosylated antibody did not give any peaks. The HPLC data indicate that the major oligosaccharides on milk-derived antibody are oligomannose, whereas cell culture-derived antibody lack oligomannose.

Concanavalin A (Con A) is a lectin that binds terminal mannosyl residues (Goldstein et al., 1965a, Biochim Biophys Acta; Goldstein et al., 1965b Biochem.). Consistent with our findings that milk-derived antibody had oligomannose, it bound to concanavalin A and was eluted with alpha-methylmannoside (**Fig. 4**). The major species in cell culture-derived material is G0F, which has terminal GlcNAc residues, was not expected to bind to concanavalin A. **Fig. 4** shows that transgenic milk-derived antibody contains oligomannose. In contrast, the aglycosylated antibody used does not bind in the same experiment.

The major species present in HEK 293 cell-derived material are fucosylated G0F (55%) and G1F (37%), with G2F as a minor species (8%). This means that the cell culture-derived antibody is completely fucosylated. The cell culture-derived antibody lacks sialic acid modification as HEK 293 cells are known to inefficiently sialylate proteins made at high levels (Chitlaru et al., 2002, Biochem J 363, 619-631).

In contrast to the large majority of the glycosylation being fucosylated on the cell-culture derived antibody, the major carbohydrate (63%) in mouse milk-derived antibody is non-fucosylated Man5. The major species from the mouse milk derived-antibody runs identically to standard Man5. G1F runs close to Man5 in the HPLC analysis but confirming that the peak is a mannose oligosaccharide was the demonstration of its Endo H sensitivity (Maley et al., 1981, J Biol Chem 256:1088-1090). After treatment with Endo H the peak seen at 36.5 minutes in the mouse milk-derived sample of antibody disappears (**Fig. 2D**). There are minor species present such as core fucose-containing G1F (16%) and G2F (21%), as well as lesser amounts of non-fucosylated G1 and Man6. The MALDI-TOF analysis of the sugars in the goat milk-derived material revealed a wider distribution of mannose-containing sugars than found in the mouse milk-derived material, most notably the presence of Man7 and Man8 indicating a deficiency in mannosidase processing. This made the quantitation of the HPLC analysis more difficult as the non-fucosylated sugars were spread out over a broader range. HPLC analysis demonstrated the presence of many EndoH sensitive peaks representing 10-20% of the total sugars (**Fig. 2**).

### Flow Cytometry Analysis

Binding of the different forms of the chimeric antibodies was evaluated by FACS analysis. 2 x 10⁵ cells were incubated with antibody at a concentration of 1 µg/ml in PBS 5% FBS. Antibody was detected with 1:100 diluted FITC-labeled goat anti-human Fc (Jackson Immuno Research Labs) and analyzed by a FACSCalibur (Becton Dickinson). The data presented in **Fig. 5** demonstrate that all the antibodies studied bound antigen similarly, regardless of the source. Controls of anti-DNP antibody did not bind, as expected.

### Surface Plasmon Resonance

Kinetics of the interaction of IgG1 with CD16a was measured by Surface plasmon resonance on a BIAcore 2000 instrument and CM5 sensor chips (BIACORE, Uppsala, Sweden). Anti-HPC4 antibodies were immobilized onto the chip using NHS/EDC coupling conditions. The antibody was at a concentration of 20-50 µg/ml at pH 5.0 to give a chip with 11,000 RU. CD16a-HPC4 was captured to this antibody surface with a 3 min. injection of 30 µg/ml protein with a flow rate of 5 µl/min in 10 mM Hepes buffer, pH 7.4, containing 0.15 M NaCl and 0.005% (v/v) P20 surfactant (HBS-P buffer, BIAcore AB) with the addition of 1 mM CaCl₂. Test antibodies were then diluted into the above binding buffer to 50 µg/ml and injected onto the captured CD16a for a minute at a flow rate of 20 µl/min. The dissociation was monitored for 3 min. The surface was then regenerated with a 3-min. injection of 5 mM EDTA made in HBS-P buffer, before the next cycle of capture-binding-regeneration. **Fig. 13** shows that milk-derived antibodies have a stronger binding than cell culture-derived antibodies. Whether this increased ADCC activity was reflected in an increased affinity to the NK cell receptor, CD16, was then determined. Surface plasmon resonance measurements were used to measure the binding of the various antibodies to immobilized CD16. The milk-derived antibodies bound better than the cell culture-antibody (**Fig. 13**).

### Cellular Assays

Human renal carcinoma cells 786-O and human embryonic kidney (HEK) 293 cells were purchased from ATCC. 786-O are adherent epithelial cells from a renal cell adenocarcinoma (Williams et al., 1978, In vitro 14: 779-786). The cells were grown in RPMI 1640 medium with 2 mM L-glutamine adjusted to contain 1.5 g/L sodium bicarbonate, 4.5 g/L glucose, 10 mM HEPES, and 1.0 mM sodium pyruvate, 90%, fetal bovine serum (FBS), 10%.

CHO cells were transfected with a pCEP4 vector expressing the CD137 coding sequence. Clones were isolated by selection with hygromycin, screened by FACS for cell surface expression, and a clone was used.

### ADCC Assay

In order to test the relative ADCC activities of milk-derived and cell culture-derived antibodies, the same antibody from different sources was prepared. Four different forms of a chimeric anti-CD137 antibody were prepared. One form was aglycosylated by mutation of Asn297 to a glutamine. This served as a negative control since aglycosylated IgGs are known not to bind to Fc receptors or to be active in ADCC (Nose et al., 1983, PNAS 80: 6632:6636). Two other forms of antibody were prepared from milk, one from mouse milk and the other from goat milk. The fourth form was from transiently transfected HEK 293 cells, a human cell line.

786-O cells were collected using 1:50 Versene (Invitrogen) and resuspended in growth medium and labeled with Na₂⁵¹CrO₄ (1 µC/µl) for 1.5 hours after which the cells were washed three times with RPMI. Effector cells were human peripheral blood mononuclear cells (PBMC) prepared as previously described by van Epps et al. (Van Epps et al., 1999, J Virol 73: 5301-5308). An effector:target ratio of 200:1 was used as the viability of PBMCs is only 40-60% after thawing. Cells were co-incubated for 12 hours at 37°C and then 25 µl of supernatant were transferred for counting. Spontaneous release was determined by incubating target cells with an equal volume of media with antibody (no PBMCs). Total radioactivity was determined by incubating target cells with an equal volume of media containing 1% Triton-X100 (Sigma). The percent specific lysis was calculated as follows: [E-S]/(T-S)]*100 where T is the total radioactivity, E is the experimental release and S is the spontaneous release which was 19±3%.

In order to test the ADCC activity of these preparations, a tumor cell line that expresses CD137 was identified. The 786-O is a renal cell carcinoma cell line that expresses low amounts of CD137 and, as it happens, HER2. To compare binding of the different forms of the chimeric anti-CD 137 antibody FACS was used. By this criterion all of the antibodies bind identically (**Fig. 5**). However, when activities were measured in the ADCC assay, the efficacies of the antibodies are grossly different. Both milk-derived proteins were active whereas the cell culture material did not have any activity, although it bound just as well (**Fig. 6**). The absence of cytotoxicity without cells (i.e., spontaneous release) demonstrates that the antibodies themselves are not killing. In **Fig. 6****,** it is shown that the molecules produced in the milk of transgenic animals have enhanced ADCC activity. This characteristic elevates the ability of transgenic milk-derived antibody to kill target cells - such as tumor cells. Controls used were anti-DNP antibody which should not bind to target cells and aglycosylated anti-CD137 which should not bind to Fc receptor.

The ADCC activity of these various preparations from CHO cells transfected with an expression vector for CD137 was also tested. Again, all of the antibody preparations bound to the transfected cells identically (**Fig. 11**). The milk-derived antibodies had twice as much activity than the cell-culture derived antibody (**Fig. 12**).

The enhanced ADCC activity is in some embodiments due to the lack of fucose on the glycosylation of the heavy chain constant region. In the mouse milk, instead of fucose, the glycosylation is mostly a precursor, Man₅GlcNAc₂. However some processing has taken place as higher mannose species are not present. The milk-derived antibody Man6 is detectable at 1420 (**Fig. 1**), while higher mannose oligosaccharides at 1582 (Man7), 1744 (Man8) and 1906 (Man9) are clearly missing, indicating that some mannosidase I mediated processing is occurring.

N-acetylglucosaminyltransferase I (GnT-I) is the key enzyme leading to Golgi alpha-mannosidase II susceptibility of the growing carbohydrate chain. This enzyme is apparently functionally limiting in the mouse mammary gland as the glycosylation block leads to accumulation of Man₅GlcNAc₂ (Li et al., 1978, J Biol Chem 253: 6426-6431). This accumulation could also be the result of the antibody not spending enough time in the medial Golgi compartment where this enzyme is localized. The inability to transfer GlcNAc to this chain prevents its cleavage by Golgi-alpha-mannosidase II that would enable further processing by N-acetylglucosaminyl-transferase II (GnT-II) and galactosyltransferase. The 1,6-fucose is added after the GnT-II modification which explains why the oligomannose structures are not fucosylated (Longmore et al., 1982, carbohy Res 100: 365-392). This oligomannose glycosylated form of IgG has been previously made in Lec1 cells, CHO cells deficient in N-acetylglucosaminyltransferase I activity (Wright et al., 1994, J Exp Med 180: 1087-1096). It was found to be defective in complement-mediated hemolysis and FcRI binding. It bound substantially more C3 of the alternative pathway for complement activation than other antibodies (Wright et al., J Immunol 160: 3393-3402).

Antibody derived from goat milk shows more heterogeneous glycosylation. Although the bulk of the material is processed G1F and G2F, the mannose-containing oligosaccharides range from Man5 to Man8 (**Fig. 1**).

Low levels of fucose depletion lead to large effects in ADCC enhancement. The heavy chains in antibodies are dimers. If the glycosylation of the heavy chains was all or none, antibodies would either all have oligomannose on both heavy chains or have processed mannose, Man3GlcNAc2, on both heavy chains; there would be no mixed dimers. The high level of ADCC suggests that the chains are independently glycosylated and that only one chain has to be deficient in fucose for enhanced activity. The antibody chains multimerize in the endoplasmic reticulum whereas the glycosylation is completed in the Golgi. With independent glycosylation of the heavy chains and 20% of the chains as high mannose, only 4% of the antibody molecules would have both chains with oligomannose and 64% with both chains containing processed mannose, whereas the remaining 32% would consist of one chain with oligomannose and the other chain of processed mannose. This would give a total of 36% of the antibody molecules having at least one oligomannose chain. Therefore it seems a single heavy chain lacking fucose is sufficient for enhanced ADCC. This is reasonable considering the asymmetric binding of the Fc receptor to the immunoglobulin molecule where the D1 region binds to one chain and the D2 region binds to the other chain, roughly (Radaev et al., 2002, Mol Immun 38: 1073-1083). Therefore fucosylation only affects binding to one domain and this is sufficient for increased affinity. A similar increase in ADCC in a previous study was observed in a case where the fucosylated antibody contained 91% fucose and the fucose-depleted antibody contained 72% fucose (Shinkawa et al., 2003, J Biol Chem 278: 3466-3473).

It was surprising that the experiment worked so well on cells with such low antigen density. It has been demonstrated that fucose removal from IgG1 could reduce the antigen amount required for ADCC activity as the result of efficient activation of NK cells. This effect probably accounts for the excellent results obtained with a cell line that express CD137 poorly.

Antibody produced in mouse milk had more activity at higher concentrations than the antibody from goat milk. The antibody preparation from goat milk may contain 10% aggregates, which can bind to the PBMCs directly without binding to target cells. This non-productive binding might block the activity of the PBMCs (Kipps et al., 1985, J Exp Med 161: 1-17).

Therefore, it has been found that transgenic milk is a good source of antibodies enhanced for ADCC activity. Antibody produced in insect cells was reported to be more effective that the same antibody produced in mammalian cells (Lang et al., 2004). Other systems have been tried but have not proved as successful. Antibody produced in *Aspergillus niger* did not have increased ADCC activity, possibly because it was a mixture of aglycosylated and glycosylated antibodies (Ward et al., 2004). Chimeric antibody produced in yeast was reported to have the same ADCC activity as that derived in cell culture (Horwitz et al., 1988). Activation of FcγIIIb also leads to secretion of cytokines.

In some embodiments the fucose-containing antibody can be separated from the non-fucosylated antibody by passing the antibody mixture over a lectin column.

The accompanying drawings, which are incorporated and constitute part of the specification, illustrate embodiments of the invention, and together with the description, serve to explain the principles of the invention.

### References

1. Anolik, J. H., et al., (2003) ARTHRITIS RHEUM 48, 455-459.
2. Anumula, K. R., and Dhume, S. T. (1998) GLYCOBIOLOGY 8, 685-694.
3. Axford, J. S., N. Sumar, et al. (1992), Changes In Normal Glycosylation Mechanisms In Autoimmune Rheumatic Disease, J. CLIN INVEST 89(3): 1021-31.
4. Baguisi A, et al. (1999), Production of Goats by Somatic Cell Nuclear Transfer, NATURE BIOTECH, 17: 456-461.
5. Cammuso, C., et al., (2000) ANIM BIOTECHNOL 11, 1-17.
6. Canfield, S. M. and S. L. Morrison (1991) The binding affinity of human IgG for its high affinity Fc receptor is determined by multiple amino acids in the CH2 domain and is modulated by the hinge region, J. EXP. MED. 173(6): 1483-91.
7. Cartron, G., et al., (2002) BLOOD 99, 754-758.
8. Chen, S.H., et al. (2000), Rejection of Disseminated Metastases of Colon Carcinoma by Synergism of IL-12 Gene Therapy And 4-1 BB Costimulation, MOL. THER., 2:39-46.
9. Chitlaru, T., et al., (2002) BIOCHEM J 363: 619-31.
10. Chitlaru, T., et al., (1998) BIOCHEM J 336: 647-58.
11. Chiu M.H. et al. (1994), In Vivo Targeting Function of N-Linked Oligosaccharides with Terminating Galactose and N-Acetylgalactosamine Residues, J. BIOL. CHEM., 269 (23):16195-202).
12. Clynes, R.A., et al., (2000) NAT MED 6, 443-46.
13. Clynes, R.A., et al., (1998) PROC NATL ACAD SCI U S A 95, 652-56.
14. Dall'Ozzo, S., et al., (2004) CANCER RES 64, 4664-69.
15. Denman, J., et al., (1991) BIOTECHNOLOGY (N Y) 9, 839-43.
16. Dorai, H., B. M. Mueller, et al. (1991), Aglycosylated Chimeric mouse/human IgG1 Antibody Retains Some Effector Function, HYBRIDOMA 10(2): 211-17.
17. Ebert, K. M., et al., (1994) BIOTECHNOLOGY (NY) 12, 699-702.
18. Edmunds, T., et al., (1998) BLOOD 91, 4561-71.
19. Foell, J. et al. (2003), CD137 Co-Stimulatory T cell Receptor Engagement Reverses Acute Disease in Lupus-Prone NZB x NZW F1 mice, J. CLIN. INVEST. 111(10), 1505-1518.
20. Fuji, S., T. Nishiura, et al. (1990), Structural Heterogeneity of Sugar Chains in Immunoglobulin G. Conformation of Immunoglobulin G Molecule and Substrate Specificities of Glycosyltransferases, J. BIOL. CHEM. 265(11): 6009-18.
21. Goldstein, I. J., et al., (1965) BIOCHIM BIOPHYS ACTA 97, 68-76.
22. Goldstein, I. J., et al., (1965) BIOCHEMISTRY 4, 876-83.
23. Gottlieb, C., et al., (1975) J BIOL CHEM 250, 3303-3309.
24. Gruel, Y., et al., (2004) BLOOD 10, 10.
25. Guinn, B.A. et al. (1999), 4-1BB Cooperates With B7-1 And B7-2 In Converting A B Cell Lymphoma Cell Line Into A Long-Lasting Antitumor Vaccine, J. IMMUNOL. 162:5003-5010.
26. Harpaz, N., and Schachter, H. (1980) J BIOL CHEM 255, 4885-4893.
27. Hong, H. J., J. W. Lee, et al. (2000), A humanized anti--4-1BB monoclonal antibody suppresses antigen-induced humoral immune response in nonhuman primates, J. IMMUNOTHER. 23(6): 613-21.
28. Horwitz, A. H., et al., (1988) PROC NATL ACAD SCI USA 85, 8678-82.
29. Jalanko, A., A. Kallio, et al. (1988), An EBV-based mammalian cell expression vector for efficient expression of cloned coding sequences, BIOCHIM BIOPHYS ACTA 949(2): 206-12.
30. James, D. C., et al., (1995) BIOTECHNOLOGY (N Y) 13, 592-596.
31. Jefferis, R., J. Lund, et al. (1995), Recognition sites on human IgG for Fc gamma receptors: the role of glycosylation, IMMUNOL LETT 44(2-3): 111-17.
32. Kasinathan P. et al. (2001), Production of Calves from G1 Fibroblasts, NATURE BIOTECH, 19: 1176-78.
33. Kerr, D.E., and Wellnitz, O., Mammary Expression of New Genes to Combat Mastitis, J. ANIM. SCI. 2003. 81:38-47 (2003).
34. Kim et al. (2001), Divergent Effects of 4-1 BB Antibodies on Antitumor Immunity and on TumorReactive T-Cell Generation, CANCER RESEARCH 61: 2031-2037.
35. Kim, J. K., M. F. Tsen, et al. (1994), Catabolism of the murine IgG1 molecule: evidence that both CH2-CH3 domain interfaces are required for persistence of IgG1 in the circulation of mice, SCAND. J. IMMUNOL. 40(4): 457-65.
36. Kipps, T. J., et al., (1985) J EXP MED 161, 1-17.
37. Knight, D. M., C. Wagner, et al. (1995), The immunogenicity of the 7E3 murine monoclonal Fab antibody fragment variable region is dramatically reduced in humans by substitution of human for murine constant regions, MOL. IMMUNOL 32(16): 1271-81.
38. Koene, H. R., et al., (1997) BLOOD 90, 1109-14.
39. Lang, P., et al., (2004) BLOOD 103, 3982-85.
40. Leatherbarrow, R. J., T. W. Rademacher, et al. (1985) Effector Functions of a Monoclonal Aglycosylated Mouse Igg2a: Binding and Activation of Complement Component C1 and Interaction with Human Monocyte Fc Receptor, MOL. IMMUNOL. 22(4): 407-15.
41. Li, Q., et al. (2003), Polarization Effects of 4-1BB during CD28 Costimulation in Generating Tumor-reactive T Cells for Cancer Immunotherapy, CANCER RESEARCH 63(10): 2546-52.
42. Li, E., and Kornfeld, S. (1978) J BIOL CHEM 253, 6426-31.
43. Lifely, M.R., Hale, C., Boyce, S., Keen, M.J. and Phillips, J, (1995) GLYCOBIOLOGY 5, 813-822.
44. LoBuglio, A. F., R. H. Wheeler, et al. (1989), Mouse/human chimeric monoclonal antibody in man: kinetics and immune response, PROC. NATL. ACAD. SCI. USA 86(11): 4220-24.
45. Louis, E., et al., (2004) ALIMENT PHARMACOL THER 19, 511-19.
46. Lund, J., N. Takahashi, et al. (1993), Control of IgG/Fc glycosylation: a comparison of oligosaccharides from chimeric human/mouse and mouse subclass immunoglobulin Gs, MOL. IMMUNOL. 30(8): 741-8.
47. Lund, J., N. Takahashi, et al. (1996), Multiple interactions of IgG with its core oligosaccharide can modulate recognition by complement and human Fc gamma receptor I and influence the synthesis of its oligosaccharide chains, J. IMMUNOL. 157(11): 4963-9.
48. Lund, J., N. Takahashi, et al. (1995), Oligosaccharide-protein interactions in IgG can modulate recognition by Fc gamma receptors, FASEB. J. 9(1): 115-9.
49. Malaise, M. G., C. Hoyoux, et al. (1990), Evidence for a role of accessible galactosyl or mannosyl residues of Fc domain in the in vivo clearance of IgG antibody-coated autologous erythrocytes in the rat, CLIN. IMMUNOL. IMMUNOPATHOL. 54(3): 469-83.
50. Maley, F., and Trimble, R. B. (1981) J BIOL CHEM 256, 1088-1090.
51. Malhotra, R., M. R. Wormald, et al. (1995), Glycosylation changes of IgG associated with rheumatoid arthritis can activate complement via the mannose-binding protein, NAT. MED. 1(3): 237-43.
52. Martinet, O., et al. (2000), Immunomodulatory gene therapy with interleukin-12 and 4-1BB ligand: long-term remission of liver metastases in a mouse model, J. NATL. CANCER Inst. 92:931-936.
53. Maynard, J. and G. Georgiou (2000), Antibody engineering, ANNUAL REV. BIOMED. Eng 2: 339-76.
54. Melero, I., et al. (1997), Monoclonal antibodies against the 4-1BB T-cell activation molecule eradicate established tumors, NAT. MED. 3:682-685. BMS.
55. Melero, I., et al. (1998), Amplification of tumor immunity by gene transfer of the co-stimulatory 4-1BB ligand: synergy with the CD28 co-stimulatory pathway, EUR. J. IMMUNOL. 28:1116-1121.
56. Mimura, Y., P. Sondermann, et al. (2001), Role of oligosaccharide residues of IgG1-Fc in Fc gamma RIIB binding, J. BIOL. CHEM. 276(49): 45539-47.
57. Miller et al. (2002), 4-1BB-Specific Monoclonal Antibody Promotes the Generation of Tumor-Specific Immune Responses by Direct Activation of CD8 T Cells in a CD40-Dependent Manner, THE JOURNAL OF IMMUNOLOGY, 169: 1792-1800. Immunex.
58. Mittler, R.S., et al. (1999), Anti-4-1BB monoclonal antibodies abrogate T cell-dependent humoral immune responses in vivo through the induction of helper T cell energy, J. EXP. MED. 190:1535.
59. Mizuochi, T., T. Taniguchi, et al. (1982), Structural and numerical variations of the carbohydrate moiety of immunoglobulin G, J. IMMUNOL. 129(5): 2016-20.
60. Mori, K., et al., (2004) BIOTECHNOL BIOENG 28, 28.
61. Niwa, R., et al., (2004) CANCER RES 64, 2127-2133.
62. Niwa, R., et al., (2005) CLIN CANCER RES 11, 2327-2336.
63. Niwa, R., et al., (2004) CLIN CANCER RES 10, 6248-6255.
64. Nose, M,. and Wigzell H. (1983), Biological Significance of Carbohydrate Chains on Monoclonal Antibodies, PROC. NATL. ACAD. SCI. U S A 80(21): 6632-36.
65. Okazaki, A., et al., (2004) J MOL BIOL 336, 1239-1249.
66. Pham, P. L., S. Perret, et al. (2003), Large-scale transient transfection of serum-free suspension-growing HEK293 EBNA1 cells: Peptone additives improve cell growth and transfection efficiency, BIOTECHNOL BIOENG 84(3): 332-42.
67. Pollock, D. P., et al., (1999) J IMMUNOL METHODS 231, 147-157.
68. Rademacher, T. W. (1993), Glycosylation as a Factor Affecting Product Consistency, BIOLOGICALS 21(2): 103-4.
69. Rademacher, T. W., S. W. Homans, et al. (1986), Immunoglobulin G as a glycoprotein, BIOCHEM Soc SYMP 51: 131-48.
70. Rademacher, T. W., R. B. Parekh, et al. (1988), The role of IgG glycoforms in the pathogenesis of rheumatoid arthritis, SPRINGER SEMIN IMMUNOPATHOL 10(2-3): 231-49.
71. Raju, T. S., et al., (2000) GLYCOBIOLOGY 10, 477-486.
72. Rothman, R. J., et al., (1989) MOL IMMUNOL. 26, 1113-23.
73. Rudd, P. M., R. J. Leatherbarrow, et al. (1991), Diversification of the IgG molecule by oligosaccharides, MOL. IMMUNOL. 28(12): 1369-78.
74. REMINGTON'S PHARMACEUTICAL SCIENCES (16th ed., Osol, A., editor, Mack, Easton Press. (1980)).
75. Sacchi, S., M. Federico, et al. (2001), Treatment of B-cell non-Hodgkin's lymphoma with anti CD 20 monoclonal antibody Rituximab, CRIT. REV. ONCOL. HEMATOL. 37(1): 13-25.
76. Sambrook et al. (1989), MOLECULAR CLONING--A LABORATORY MANUAL, Cold Spring Harbor Laboratory Press, (2nd Edition).
77. Shen, E. S., G. M. Cooke, et al. (1995), Improved expression cloning using reporter genes and Epstein-Barr virus ori-containing vectors, GENE 156(2): 235-9.
78. Shields, R. L., et al., (2002) J BIOL CHEM 277, 26733-26740.
79. Shinkawa, T., et al., (2002) J BIOL CHEM 8, 8.
80. Simmons, L. C., D. Reilly, et al. (2002), Expression of full-length immunoglobulins in Escherichia coli: rapid and efficient production of aglycosylated antibodies, J. IMMUNOL. METHODS 263(1-2): 133-47.
81. Stanley, P. (1984), Glycosylation Mutants of Animal Cells, ANNUAL REV. GENET. 18: 525-52.
82. Stanley, P., et al., (1975) PROC NATL ACAD SCI U S A 72, 3323-27.
83. Strome S.E. et al. (2002), Strategies for antigen loading of dendritic cells to enhance the antitumor immune response, CANCER RESEARCH 62:1884-89.
84. Sumar, N., K. B. Bodman, et al. (1990), Analysis of glycosylation changes in IgG using lectins, J. IMMUNOL. METHODS 131(1): 127-36.
85. Sutton, B. J. and D. C. Phillips (1983), The three-dimensional structure of the carbohydrate within the Fc fragment of immunoglobulin G, BIOCHEM SOC TRANS 11(Pt 2): 130-2.
86. Sun, Y. et al. (2002), Costimulatory molecule-targeted antibody therapy of a spontaneous autoimmune disease, NAT MED. 8(12), 1405-13.
87. Sun, Y. et al. (2002), Administration of agonistic anti-4-1BB monoclonal antibody leads to the amelioration of experimental autoimmune encephalomyelitis, J. IMMUNOL. 168(3), 1457-65.
88. Takeuchi, T., et al., (1999) AUTOIMMUNITY 31, 265-271.
89. Tamamori, Y., et al., (2002) INT J ONCOL 21, 649-654.
90. Tandai, M., T. Endo, et al. (1991), Structural study of the sugar moieties of monoclonal antibodies secreted by human-mouse hybridoma, ARCH. BIOCHEM. BIOPHYS.291(2): 339-48.
91. Tao, M. H. and S. L. Morrison (1989), Studies of aglycosylated chimeric mouse-human IgG. Role of carbohydrate in the structure and effector functions mediated by the human IgG constant region, J. IMMUNOL. 143(8): 2595-601.
92. Tao, M. H., R. I. Smith, et al. (1993), Structural features of human immunoglobulin G that determine isotype-specific differences in complement activation, J. EXP. MED. 178(2): 661-67.
93. Tarentino, A. L., et al., (1974) J BIOL CHEM 249, 818-24.
94. Treon, S. P., et al., (2005) J CLIN ONCOL 23, 474-81.
95. Tsuchiya, N., T. et al. (1989), Effects of galactose depletion from oligosaccharide chains on immunological activities of human IgG, J. RHEUMATOL. 16(3): 285-90.
96. Van Epps, H. L., Schmaljohn, C. S., and Ennis, F. A. (1999) J VIROL 73, 5301-5308.
97. Walker, M. R., J. Lund, et al. (1989), Aglycosylation of human IgG1 and IgG3 monoclonal antibodies can eliminate recognition by human cells expressing Fc gamma RI and/or Fc gamma RII receptors, BIOCHEM J. 259(2): 347-53.
98. Ward, M., et al., (2004) APPL ENVIRON MICROBIOL 70, 2567-76.
99. White, K. D., M. B. Frank, et al. (1996), Effect of immunoglobulin variable region structure on C3b and C4b deposition, MOL. IMMUNOL. 33(9): 759-68.
100. Wilcox R.A., et al. (2002), Provision of antigen and CD137 signaling breaks immunological ignorance, promoting regression of poorly immunogenic tumors, J. CLIN. INVEST. 109:651-59.
101. Wilcox R.A. et al. (2002), Signaling through NK cell-associated CD137 promotes both helper function for CD8+ cytolytic T lymphocytes and responsiveness to interleukin-2, J. IMMUNOL. 169:4230-36.
102. Williams, R. D., et al., (1978) IN VITRO 14, 779-786.
103. Wilmut I, et al. (Oct 10, 2002), Somatic Cell Nuclear Transfer, NATURE 419(6907):583-86.
104. Wilmut I, et al. (Feb 27, 1997), Viable Offspring Derived From Fetal and Adult Mammalian Cells, NATURE 385(6619):810-3.
105. Wolff, E. A., et al., (1993) CANCER RES 53, 2560-65.
106. Wright, A. and S. L. Morrison (1994), Effect of altered CH2-associated Carbohydrate Structure On The Functional Properties And In Vivo Fate Of Chimeric Mouse-Human Immunoglobulin G1, J. EXP. MED. 180(3): 1087-96.
107. Wright, A., and Morrison, S. L. (1998) J IMMUNOL 160, 3393-3402.
108. Wu, J., et al,. (1997) J CLIN INVEST 100, 1059-1070.
109. Yamane-Ohnuki, N., et al., (2004) BIOTECHNOL. BIOENG. 87, 614-22.
110. Zhou, Q., et al., (2005) J. BIOTECHNOL. 117, 57-72.
111. Zhu G., et al. (2001), Progressive depletion of peripheral B lymphocytes in 4-1BB (CD137) ligand/I-Eβ transgenic mice, J. IMMUNOL. 167:2671-6.
112. Zou X, et al. (2002), Generation of Cloned Goats (Capra Hircus) from Transfected Foetal Fibroblast Cells, the Effect of Donor Cell Cycle, MOL REPROD DEV.; 61: 164-172.
   Chen et al., 20050013811

## Claims

1. Use of non-human mammalian mammary epithelial cells for enhancing the binding of the Fc region of an IgG antibody or IgG antibodies to an FcγRIII receptor compared to a cell culture-derived antibody or antibodies, wherein said cells are of a non-human mammal engineered to express the antibody in its milk and wherein the glycosylation pattern of the antibody is modified by producing the antibody or antibodies in the cells.

2. The use of claim 1, wherein the antibody or antibodies are of the isotype IgG1 or IgG2.

3. The use of any one of claims 1 or 2, wherein the antibody is modified such that at least one chain of the antibody does not contain fucose.

4. The use of any one of claims 1-3, wherein the antibody is modified such that the antibody contains an oligomannose or an additional oligomannose.

5. The use of any one of claims 1-4, wherein the antibody is modified such that the carbohydrates of the antibody exhibit a high mannose glycosylation pattern.

6. The use of any one of claims 1-5, wherein the antibody is modified such that at least one chain of the antibody is oligomannose-containing and non-fucosylated.

7. The use of any one of claims 1-6, wherein the antibody is modified such that the major carbohydrate of the antibody is non-fucosylated, and is preferably a non-fucosylated oligomannose, still preferably non-fucosylated Man5.

8. The use of any one of claims 1-7, wherein the antibody is modified such that less than 40% of the carbohydrates of the antibody contain fucose.

9. The use of any one of claims 1-8, wherein the antibodies are modified such that at least 30% of the antibodies have at least one oligomannose.

10. The use of any one of claims 1-9, wherein at least 60% of the carbohydrates of the antibodies are a non-fucosylated oligomannose and less than 40% of the carbohydrates of the antibodies are fucose-containing.

11. The use of any one of claims 1-10, wherein the mammary epithelial cells are cells from a goat, sheep, bison, camel, cow, pig, rabbit, buffalo, horse, rat, mouse or Ilama.

12. The use of any one of claims 1-11, wherein the antibody is a chimeric antibody, humanized antibody, fully human antibody, or an antibody fragment.

13. The use of any one of claims 1-12, wherein the antibody is a full-length antibody which preferably comprises a heavy chain and a light chain.

14. The use of any one of claims 1-13, wherein the antibody is an anti CD137 antibody.

## Patentansprüche

1. Verwendung von Mammaepithelzellen eines nicht menschlichen Säugetiers zur Verstärkung der Bindung der Fc-Region eines IgG-Antikörpers oder von IgG-Antikörpern an den FcγRIII-Rezeptor im Vergleich zu einem Zellkultur gewonnenen Antikörper oder Antikörpern, wobei die Zellen von einem nicht menschlichen Säugetier stammen und so technisch verändert sind, dass der Antikörper in dessen Milch exprimiert wird und wobei das Glykosylierungsmuster des Antikörpers durch die Herstellung des Antikörpers oder der Antikörper in den Zellen modifiziert wird.

2. Die Verwendung nach Anspruch 1, wobei der Antikörper oder die Antikörper vom Isotyp IgG1 oder IgG2 sind.

3. Die Verwendung nach einem der Ansprüche 1 oder 2, wobei der Antikörper derart modifiziert ist, dass mindestens eine Kette des Antikörpers keine Fucose enthält.

4. Die Verwendung nach einem der Ansprüche 1-3, wobei der Antikörper derart modifiziert ist, dass der Antikörper eine Oligomannose oder eine zusätzliche Oligomannose enthält.

5. Die Verwendung nach einem der Ansprüche 1-4, wobei der Antikörper derart modifiziert ist, dass die Kohlenhydrate des Antikörpers ein hohes Mannoseglykosylierungsmuster aufweisen.

6. Die Verwendung nach einem der Ansprüche 1-5, wobei der Antikörper derart modifiziert ist, dass mindestens eine Kette des Antikörpers oligomannosehaltig und nicht fucosyliert ist.

7. Die Verwendung nach einem der Ansprüche 1-6, wobei der Antikörper derart modifiziert ist, dass das Hauptkohlenhydrat des Antikörpers nicht fucosyliert ist und vorzugsweise eine nicht fucosylierte Oligomannose ist, noch bevorzugter nicht fucosylierte Man5 ist.

8. Die Verwendung nach einem der Ansprüche 1-7, wobei der Antikörper derart modifiziert ist, dass weniger als 40% der Kohlenhydrate des Antikörpers Fucose enthalten.

9. Die Verwendung nach einem der Ansprüche 1-8, wobei die Antikörper derart modifiziert sind, dass mindestens 30% der Antikörper mindestens eine Oligomannose haben.

10. Die Verwendung nach einem der Ansprüche 1-9, wobei mindestens 60% der Kohlenhydrate der Antikörper nicht fucosylierte Oligomannose sind und weniger als 40% der Kohlenhydrate der Antikörper fucosehaltig sind.

11. Die Verwendung nach einem der Ansprüche 1-10, wobei die Mammaepithelzellen Zellen einer Ziege, eines Schafs, eines Bisons, eines Kamels, einer Kuh, eines Schweins, eines Kaninchens, eines Büffels, eines Pferds, einer Ratte, einer Maus oder eines Lamas sind.

12. Die Verwendung nach einem der Ansprüche 1-11, wobei der Antikörper ein chimärer Antikörper, ein humanisierter Antikörper, ein vollständig humaner Antikörper oder ein Antikörperfragment ist.

13. Die Verwendung nach einem der Ansprüche 1-12, wobei der Antikörper ein Antikörper voller Länge ist, der vorzugsweise eine schwere und eine leichte Kette umfasst.

14. Die Verwendung nach einem der Ansprüche 1-13, wobei der Antikörper ein anti-CD137-Antikörper ist.

## Revendications

1. Utilisation de cellules épithéliales mammaires de mammifères non humains pour renforcer la liaison de la région Fc d'un anticorps IgG ou d'anticorps IgG à un récepteur FcγRIII par comparaison à un anticorps ou à des anticorps dérivés de culture cellulaire, où lesdites cellules proviennent d'un mammifère non humain manipulé génétiquement pour exprimer l'anticorps dans son lait et où le profil de glycosylation de l'anticorps est modifié par la production de l'anticorps ou des anticorps dans les cellules.

2. Utilisation selon la revendication 1, où l'anticorps ou les anticorps sont de l'isotype IgG1 ou IgG2.

3. Utilisation selon l'une quelconque des revendications 1 ou 2, où l'anticorps est modifié de sorte qu'au moins une chaîne de l'anticorps ne contient pas de fucose.

4. Utilisation selon l'une quelconque des revendications 1 à 3, où l'anticorps est modifié de sorte que l'anticorps contient un oligomannose ou un oligomannose supplémentaire.

5. Utilisation selon l'une quelconque des revendications 1 à 4, où l'anticorps est modifié de sorte que les hydrates de carbone de l'anticorps présentent un profil de glycosylation à forte teneur en mannose.

6. Utilisation selon l'une quelconque des revendications 1 à 5, où l'anticorps est modifié de sorte qu'au moins une chaîne de l'anticorps contient un oligomannose et est non fucosylée.

7. Utilisation selon l'une quelconque des revendications 1 à 6, où l'anticorps est modifié de sorte que l'hydrate de carbone majeur de l'anticorps est non fucosylé, et est de préférence un oligomannose non fucosylé, de préférence encore un Man5 non fucosylé.

8. Utilisation selon l'une quelconque des revendications 1 à 7, où l'anticorps est modifié de sorte que moins de 40 % des hydrates de carbone de l'anticorps contiennent un fucose.

9. Utilisation selon l'une quelconque des revendications 1 à 8, où les anticorps sont modifiés de sorte qu'au moins de 30 % des anticorps ont au moins un oligomannose.

10. Utilisation selon l'une quelconque des revendications 1 à 9, où au moins 60 % des hydrates de carbone des anticorps sont un oligomannose non fucosylé et moins de 40 % des hydrates de carbone des anticorps contiennent un fucose.

11. Utilisation selon l'une quelconque des revendications 1 à 10, où les cellules épithéliales mammaires sont des cellules provenant d'une chèvre, d'un mouton, d'un bison, d'un chameau, d'une vache, d'un porc, d'un lapin, d'un buffle, d'un cheval, d'un rat, d'une souris ou d'un lama.

12. Utilisation selon l'une quelconque des revendications 1 à 11, où l'anticorps est un anticorps chimérique, un anticorps humanisé, un anticorps complètement humain, ou un fragment d'anticorps.

13. Utilisation selon l'une quelconque des revendications 1 à 12, où l'anticorps est un anticorps entier qui comprend de préférence une chaîne lourde et une chaîne légère.

14. Utilisation selon l'une quelconque des revendications 1 à 13, où l'anticorps est un anticorps anti-CD137.
